# NEW EUROPEAN PATENT SPECIFICATION

(11) **EP 2 864 447 B3**
(45) Date of publication of this specification: **17.07.2019**
(45) Mention of the grant of the patent: 06.12.2017
(21) Application number: 13733207.8
(22) Date of filing: 20.06.2013
(51) Int. Cl.: C10G 3/00, C11B 3/02, C11C 3/00, A61K 8/37, A61K 8/49, A61Q 19/10, C11B 3/00, C11C 3/10

(54) **NATURAL OIL METATHESIS COMPOSITIONS**
METATHESEZUSAMMENSETZUNGEN NATÜRLICHER ÖLE
COMPOSITIONS OBTENUES PAR MÉTATHÈSE D'HUILE NATURELLE

(30) Priority: 20.06.2012 US 201261662318 P; 14.03.2013 US 201361781892 P
(43) Date of publication of application: 29.04.2015
(73) Proprietor: Elevance Renewable Sciences, Inc., Woodridge, IL 60517 (US)
(72) Inventor: COHEN, Steven A., Naperville, IL 60540 (US); MORIE-BEBEL, M., Michelle, Naperville, IL 60540 (US); ILSEMAN, Alexander D., Chicago, IL 60616 (US); BERGMANN, Benjamin, Anderson, SC 29625 (US); DIBIASE, Stephen A., River Forest, IL 60305 (US); CHRISTENSEN, S., Alexander, Northwoods, IL 60185 (US)
(74) Representative: Murgitroyd & Company
(86) International application number: PCT/US2013/046735
(87) International publication number: WO 2013/192384

(56) References cited:
- WO-A1-2010/062958
- WO-A1-2012/006324
- WO-A2-2011/046872

## Description

### CROSS-REFERENCE TO RELATED APPLICATIONS

### BACKGROUND

In recent years, there has been an increased demand for environmentally friendly techniques for manufacturing materials typically derived from petroleum sources. In view of the non-renewable nature of petroleum, it is highly desirable to provide non-petroleum alternatives for material manufacturing biofuels, waxes, plastics, cosmetics, personal care items, and the like. One of the methods to manufacture such materials is through generating compositions through the metathesis of natural oil feedstocks, such as vegetable and seed-based oils. WO 2011/046872 discloses a metathesized natural oil composition.

### SUMMARY OF INVENTION

The present invention relates to a metathesized natural oil composition as claimed hereinafter. Preferred embodiments of the invention are set forth in the dependent claims. Associated methods are also described herein to aid the understanding of the invention, but these do not form part of the claimed invention. Examples or embodiments described herein which do not fall within the definition of the claims do not form part of the present invention.

### BRIEF DESCRIPTION OF THE DRAWINGS

FIG. 1 depicts a schematic diagram of a process to produce a metathesized natural oil product and a transesterified product from a natural oil.
FIG. 2 depicts a mass spectrum of a metathesized natural oil product.
FIG. 3 depicts a mass spectrum of a metathesized natural oil product.
FIG. 4 depicts a mass spectrum of a metathesized natural oil product.
FIG. 5 depicts a mass spectrum of a metathesized natural oil product.
FIG. 6 depicts a gel permeation chromatogram of metathesized natural oil products.
FIG. 7 depicts the dynamic viscosity as a function of time for metathesized natural oil products.
FIG. 8 depicts a gel permeation chromatogram overlay of kinetic studies of metathesized natural oil products.
FIG. 9 depicts viscosity as a function of blending two different metathesized natural oil products.
FIG. 10 depicts the kinetics of metathesized natural oil products.
FIG. 11 depicts a gel permeation chromatogram overlay of kinetic studies of metathesized natural oil products.
FIG. 12 depicts a gel permeation chromatogram overlay of kinetic studies of metathesized natural oil products.
FIG. 13 depicts a gel permeation chromatogram of a metathesized natural oil product.
FIG. 14 depicts a gel permeation chromatogram of a metathesized natural oil product.
FIG. 15 depicts a gel permeation chromatogram of a metathesized natural oil product.
FIG. 16 depicts a gel permeation chromatogram of a metathesized natural oil product.

### DETAILED DESCRIPTION

The terms "natural oils," "natural feedstocks," or "natural oil feedstocks" may refer to oils derived from plants or animal sources. The term "natural oil" includes natural oil derivatives, unless otherwise indicated. The terms also include modified plant or animal sources (e.g., genetically modified plant or animal sources), unless indicated otherwise. Examples of natural oils include, but are not limited to, vegetable oils, algae oils, fish oils, animal fats, tall oils, derivatives of these oils, combinations of any of these oils, and the like. Representative non-limiting examples of vegetable oils include canola oil, rapeseed oil, coconut oil, corn oil, cottonseed oil, olive oil, palm oil, peanut oil, safflower oil, sesame oil, soybean oil ("SBO"), sunflower oil, linseed oil, palm kernel oil, tung oil, jatropha oil, mustard oil, pennycress oil, camelina oil, and castor oil. Representative non-limiting examples of animal fats include lard, tallow, poultry fat, yellow grease, and fish oil. Tall oils are by-products of wood pulp manufacture.

The term "natural oil derivatives" refers to derivatives thereof derived from natural oil. The methods used to form these natural oil derivatives may include one or more of addition, neutralization, overbasing, saponification, transesterification, esterification, amidification, hydrogenation, isomerization, oxidation, alkylation, acylation, sulfurization, sulfonation, rearrangement, reduction, fermentation, pyrolysis, hydrolysis, liquefaction, anaerobic digestion, hydrothermal processing, gasification or a combination of two or more thereof. Examples of natural derivatives thereof may include carboxylic acids, gums, phospholipids, soapstock, acidulated soapstock, distillate or distillate sludge, fatty acids, fatty acid esters, as well as hydroxy substituted variations thereof, including unsaturated polyol esters. In the present disclosure, the natural oil derivative may comprise an unsaturated carboxylic acid having from about 5 to about 30 carbon atoms, having one or more carbon-carbon double bonds in the hydrocarbon (alkene) chain. The natural oil derivative may also comprise an unsaturated fatty acid alkyl (e.g., methyl) ester derived from a glyceride of natural oil. For example, the natural oil derivative may be a fatty acid methyl ester ("FAME") derived from the glyceride of the natural oil. In some embodiments, a feedstock includes canola or soybean oil, as a non-limiting example, refined, bleached, and deodorized soybean oil (i.e., RBD soybean oil).

The term "low-molecular-weight olefin" may refer to any one or combination of unsaturated straight, branched, or cyclic hydrocarbons in the C₂ to C₁₄ range. Low-molecular-weight olefins include "alpha-olefins" or "terminal olefins," wherein the unsaturated carbon-carbon bond is present at one end of the compound. Low-molecular-weight olefins may also include dienes or trienes. Examples of low-molecular-weight olefins in the C₂ to C₆ range include, but are not limited to: ethylene, propylene, 1-butene, 2-butene, isobutene, 1-pentene, 2-pentene, 3-pentene, 2-methyl-1-butene, 2-methyl-2-butene, 3-methyl-1-butene, cyclopentene, 1-hexene, 2-hexene, 3-hexene, 4-hexene, 2-methyl-1-pentene, 3-methyl-1-pentene,4-methyl-1-pentene,2-methyl-2-pentene, 3-methyl-2-pentene,4-methyl-2-pentene,2-methyl-3-pentene, and cyclohexene. Other possible low-molecular-weight olefins include styrene and vinyl cyclohexane. Disclosed herein is the use of a mixture of olefins, the mixture comprising linear and branched low-molecular-weight olefins in the C₄-C₁₀ range,the use of a mixture of linear and branched C₄ olefins (i.e., combinations of: 1-butene, 2-butene, and/or isobutene) or a higher range of C₁₁-C₁₄ may be used.

The term "metathesis monomer" refers to a single entity that is the product of a metathesis reaction which comprises a molecule of a compound with one or more carbon-carbon double bonds which has undergone an alkylidene unit interchange via one or more of the carbon-carbon double bonds either within the same molecule (intramolecular metathesis) and/or with a molecule of another compound containing one or more carbon-carbon double bonds such as an olefin (intermolecular metathesis).

The term "metathesis dimer" refers to the product of a metathesis reaction wherein two reactant compounds, which can be the same or different and each with one or more carbon-carbon double bonds, are bonded together via one or more of the carbon-carbon double bonds in each of the reactant compounds as a result of the metathesis reaction.

The term "metathesis trimer" refers to the product of one or more metathesis reactions wherein three molecules of two or more reactant compounds, which can be the same or different and each with one or more carbon-carbon double bonds, are bonded together via one or more of the carbon-carbon double bonds in each of the reactant compounds as a result of the one or more metathesis reactions, the trimer containing three bonded groups derived from the reactant compounds.

The term "metathesis tetramer" refers to the product of one or more metathesis reactions wherein four molecules of two or more reactant compounds, which can be the same or different and each with one or more carbon-carbon double bonds, are bonded together via one or more of the carbon-carbon double bonds in each of the reactant compounds as a result of the one or more metathesis reactions, the tetramer containing four bonded groups derived from the reactant compounds.

The term "metathesis pentamer" refers to the product of one or more metathesis reactions wherein five molecules of two or more reactant compounds, which can be the same or different and each with one or more carbon-carbon double bonds, are bonded together via one or more of the carbon-carbon double bonds in each of the reactant compounds as a result of the one or more metathesis reactions, the pentamer containing five bonded groups derived from the reactant compounds.

The term "metathesis hexamer" refers to the product of one or more metathesis reactions wherein six molecules of two or more reactant compounds, which can be the same or different and each with one or more carbon-carbon double bonds, are bonded together via one or more of the carbon-carbon double bonds in each of the reactant compounds as a result of the one or more metathesis reactions, the hexamer containing six bonded groups derived from the reactant compounds.

The term "metathesis heptamer" refers to the product of one or more metathesis reactions wherein seven molecules of two or more reactant compounds, which can be the same or different and each with one or more carbon-carbon double bonds, are bonded together via one or more of the carbon-carbon double bonds in each of the reactant compounds as a result of the one or more metathesis reactions, the heptamer containing seven bonded groups derived from the reactant compounds.

The term "metathesis octamer" refers to the product of one or more metathesis reactions wherein eight molecules of two or more reactant compounds, which can be the same or different and each with one or more carbon-carbon double bonds, are bonded together via one or more of the carbon-carbon double bonds in each of the reactant compounds as a result of the one or more metathesis reactions, the octamer containing eight bonded groups derived from the reactant compounds.

The term "metathesis nonamer" refers to the product of one or more metathesis reactions wherein nine molecules of two or more reactant compounds, which can be the same or different and each with one or more carbon-carbon double bonds, are bonded together via one or more of the carbon-carbon double bonds in each of the reactant compounds as a result of the one or more metathesis reactions, the nonamer containing nine bonded groups derived from the reactant compounds.

The term "metathesis decamer" refers to the product of one or more metathesis reactions wherein ten molecules of two or more reactant compounds, which can be the same or different and each with one or more carbon-carbon double bonds, are bonded together via one or more of the carbon-carbon double bonds in each of the reactant compounds as a result of the one or more metathesis reactions, the decamer containing ten bonded groups derived from the reactant compounds.

The term "metathesis oligomer" refers to the product of one or more metathesis reactions wherein two or more molecules (e.g., 2 to about 10, or 2 to about 4) of two or more reactant compounds, which can be the same or different and each with one or more carbon-carbon double bonds, are bonded together via one or more of the carbon-carbon double bonds in each of the reactant compounds as a result of the one or more metathesis reactions, the oligomer containing a few (e.g., 2 to about 10, or 2 to about4) bonded groups derived from the reactant compounds. In the present disclosure, the term "metathesis oligomer" may include metathesis reactions wherein greater than ten molecules of two or more reactant compounds, which can be the same or different and each with one or more carbon-carbon double bonds, are bonded together via one or more of the carbon-carbon double bonds in each of the reactant compounds as a result of the one or more metathesis reactions, the oligomer containing greater than ten bonded groups derived from the reactant compounds.

As used herein, the terms "metathesize" and "metathesizing" may refer to the reacting of a natural oil feedstock in the presence of a metathesis catalyst to form a metathesized natural oil product comprising a new olefinic compound and/or esters. Metathesizing may refer to cross-metathesis (a.k.a. co-metathesis), self-metathesis, ring-opening metathesis, ring-opening metathesis polymerizations ("ROMP"), ring-closing metathesis ("RCM"), and acyclic diene metathesis ("ADMET"). As a non-limiting example, metathesizing may refer to reacting two triglycerides present in a natural feedstock (self-metathesis) in the presence of a metathesis catalyst, wherein each triglyceride has an unsaturated carbon-carbon double bond, thereby forming an oligomer having a new mixture of olefins and esters that may comprise one or more of: metathesis monomers, metathesis dimers, metathesis trimers, metathesis tetramers, metathesis pentamers, and higher order metathesis oligomers (e.g., metathesis hexamers, metathesis, metathesis heptamers, metathesis octamers, metathesis nonamers, metathesis decamers, and higher than metathesis decamers and above).

Metathesis is a catalytic reaction generally known in the art that involves the interchange of alkylidene units among compounds containing one or more double bonds (e.g., olefinic compounds) via the formation and cleavage of the carbon-carbon double bonds. Metathesis may occur between two like molecules (often referred to as self-metathesis) and/or it may occur between two different molecules (often referred to as cross-metathesis). Self-metathesis may be represented schematically as shown in Equation A below.

Equation A R¹-CH=CH-R² + R¹-CH=CH-R² ↔ R¹-CH=CH-R¹ + R²-CH=CH-R²

wherein R¹ and R² are organic groups.
Cross-metathesis may be represented schematically as shown in Equation B below.

Equation B R¹-CH=CH-R² + R³-CH=CH-R⁴ ↔ R¹-CH=CH-R³ + R¹-CH=CH-R⁴ + R²-CH=CH-R³ + R²-CH=CH-R⁴ + R¹-CH=CH-R¹ + R²-CH=CH-R² + R³-CH=CH-R³ + R⁴-CH=CH-R⁴

wherein R¹, R², R³, and R⁴ are organic groups.

The metathesis reaction of the natural oil feedstock having polyol esters (of fatty acids) results in the oligomerization of the natural oil feedstock having a mixture of olefins and esters that may comprise one or more of: metathesis monomers, metathesis dimers, metathesis trimers, metathesis tetramers, metathesis pentamers, and higher order metathesis oligomers (e.g., metathesis hexamers, metathesis heptamers, metathesis octamers, metathesis nonamers, metathesis decamers, and higher than metathesis decamers).

Natural oils of the type described herein typically are composed of triglycerides of fatty acids. These fatty acids may be either saturated, monounsaturated or polyunsaturated and contain varying chain lengths ranging from C₈ to C₃₀. The most common fatty acids include saturated fatty acids such as lauric acid (dodecanoic acid), myristic acid (tetradecanoic acid), palmitic acid (hexadecanoic acid), stearic acid (octadecanoic acid), arachidic acid (eicosanoic acid), and lignoceric acid (tetracosanoic acid); unsaturated acids include such fatty acids as palmitoleic (a C16 acid), and oleic acid (a C18 acid); polyunsaturated acids include such fatty acids as linoleic acid (a di-unsaturated C18 acid), linolenic acid (a tri-unsaturated C18 acid), and arachidonic acid (a tetra-unsubstituted C20 acid). The natural oils are further comprised of esters of these fatty acids in random placement onto the three sites of the trifunctional glycerine molecule. Different natural oils will have different ratios of these fatty acids, and within a given natural oil there is a range of these acids as well depending on such factors as where a vegetable or crop is grown, maturity of the vegetable or crop, the weather during the growing season, etc. Thus, it is difficult to have a specific or unique structure for any given natural oil, but rather a structure is typically based on some statistical average. For example soybean oil contains a mixture of stearic acid, oleic acid, linoleic acid, and linolenic acid in the ratio of 15:24:50:11, and an average number of double bonds of 4.4-4.7 per triglyceride. One method of quantifying the number of double bonds is the iodine value (IV) which is defined as the number of grams of iodine that will react with 100 grams of vegetable oil. Therefore for soybean oil, the average iodine value range is from 120-140. Soybean oil may comprises about 95% by weight or greater (e.g., 99% weight or greater) triglycerides of fatty acids. Major fatty acids in the polyol esters of soybean oil include saturated fatty acids, as a non-limiting example, palmitic acid (hexadecanoic acid) and stearic acid (octadecanoic acid), and unsaturated fatty acids, as a non-limiting example, oleic acid (9-octadecenoic acid), linoleic acid (9, 12-octadecadienoic acid), and linolenic acid (9,12,15-octadecatrienoic acid).

When a polyol ester comprises molecules having more than one carbon-carbon double bond, self-metathesis may result in oligomerization or polymerization of the unsaturates in the starting material. For example, Equation C depicts metathesis oligomerization of a representative species (e.g., a polyol ester) having more than one carbon-carbon double bond. In Equation C, the self-metathesis reaction results in the formation of metathesis dimers, metathesis trimers, and metathesis tetramers. Although not shown, higher order oligomers such as metathesis pentamers, hexamers, heptamers, octamers, nonamers, decamers, and higher than decamers, and mixtures of two or more thereof, may also be formed. The number of metathesis repeating units or groups in the metathesized natural oil may range from 1 to about 100, or from 2 to about 50, or from 2 to about 30, or from 2 to about 10, or from 2 to about 4. The molecular weight of the metathesis dimer may be greater than the molecular weight of the unsaturated polyol ester from which the dimer is formed. Each of the bonded polyol ester molecules may be referred to as a "repeating unit or group." Typically, a metathesis trimer may be formed by the cross-metathesis of a metathesis dimer with an unsaturated polyol ester. Typically, a metathesis tetramer may be formed by the cross-metathesis of a metathesis trimer with an unsaturated polyol ester or formed by the cross-metathesis of two metathesis dimers.

Equation C R¹-HC=CH-R²-HC=CH-R³ + R¹-HC=CH-R²-HC=CH-R³ ↔ R¹-HC=CH-R²-HC=CH-R²-HC=CH-R³ + (other products) (metathesis dimer) R¹-R²-HC=CH-R²-HC=CH-R³ + R¹-HC=CH-R²-HC=CH-R³ ↔ R¹-HC=CH-R²-HC=CH-R²-HC=CH-R²-HC=CH-R³ + (other products) (metathesis trimer) R¹-HC=CH-R²-HC=CH-R²-HC=CH-R²-HC=CH-R³ + R¹-HC=CH-R²-HC=CH-R³ ↔ R¹-HC=CH-R²-HC=CH-R²-HC=CH-R²-HC=CH-R²-HC=CH-R³ + (other products) (metathesis tetramer)

where R¹, R², and R³ are organic groups.

As noted, the self-metathesis of the natural oil occurs in the presence of a metathesis catalyst. As stated previously, the term "metathesis catalyst" includes any catalyst or catalyst system that catalyzes a metathesis reaction. Any known metathesis catalyst may be used, alone or in combination with one or more additional catalysts. Suitable homogeneous metathesis catalysts include combinations of a transition metal halide or oxo-halide (e.g., WOCl₄ or WCl₆) with an alkylating cocatalyst (e.g., Me₄Sn), or alkylidene (or carbene) complexes of transition metals, particularly Ru, Mo, or W. These include first and second-generation Grubbs catalysts, Grubbs-Hoveyda catalysts, and the like. Suitable alkylidene catalysts have the general structure:

M[X¹X²L¹L²(L³)ₙ]=Cₘ=C(R¹)R²

where M is a Group 8 transition metal, L¹, L², and L³ are neutral electron donor ligands, n is 0 (such that L³ may not be present) or 1, m is 0, 1, or 2, X¹ and X² are anionic ligands, and R¹ and R² are independently selected from H, hydrocarbyl, substituted hydrocarbyl, heteroatom-containing hydrocarbyl, substituted heteroatom-containing hydrocarbyl, and functional groups. Any two or more of X¹, X², L¹, L², L³, R¹ and R² can form a cyclic group and any one of those groups can be attached to a support.

First-generation Grubbs catalysts fall into this category where m=n=0 and particular selections are made for n, X¹, X², L¹, L², L³, R¹ and R² as described in U.S. Pat. Appl. Publ. No. 2010/0145086, the teachings of which related to all metathesis catalysts.

Second-generation Grubbs catalysts also have the general formula described above, but L¹ is a carbene ligand where the carbene carbon is flanked by N, O, S, or P atoms, preferably by two N atoms. Usually, the carbene ligand is part of a cyclic group. Examples of suitable second-generation Grubbs catalysts also appear in the '086 publication.

In another class of suitable alkylidene catalysts, L¹ is a strongly coordinating neutral electron donor as in first- and second-generation Grubbs catalysts, and L² and L³ are weakly coordinating neutral electron donor ligands in the form of optionally substituted heterocyclic groups. Thus, L² and L³ are pyridine, pyrimidine, pyrrole, quinoline, thiophene, or the like.

In yet another class of suitable alkylidene catalysts, a pair of substituents is used to form a bi- or tridentate ligand, such as a biphosphine, dialkoxide, or alkyldiketonate. Grubbs-Hoveyda catalysts are a subset of this type of catalyst in which L² and R² are linked. Typically, a neutral oxygen or nitrogen coordinates to the metal while also being bonded to a carbon that is α-, β-, or γ- with respect to the carbene carbon to provide the bidentate ligand. Examples of suitable Grubbs-Hoveyda catalysts appear in the '086 publication.

The structures below provide just a few illustrations of suitable catalysts that may be used:

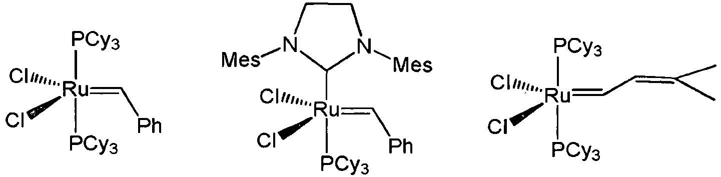

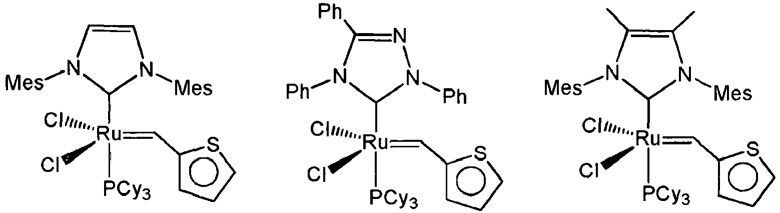

Heterogeneous catalysts suitable for use in the self- or cross-metathesis reaction include certain rhenium and molybdenum compounds as described, e.g., by J.C. Mol in Green Chem. 4 (2002) 5 at pp. 11-12. Particular examples are catalyst systems that include Re₂O₇ on alumina promoted by an alkylating cocatalyst such as a tetraalkyl tin lead, germanium, or silicon compound. Others include MoCl₃ or MoCl₅ on silica activated by tetraalkyltins.

For additional examples of suitable catalysts for self- or cross-metathesis, see U.S. Pat. Nos. 4,545,941, 5,312,940, 5,342,909, 5,710,298, 5,728,785, 5,728,917, 5,750,815, 5,831,108, 5,922,863, 6,306,988, 6,414,097, 6,696,597,6,794,534,7,102,047,7,378,528, and U.S. Pat. Appl. Publ. No. 2009/0264672 A1, and PCT/US2008/009635, pp. 18-47. A number of metathesis catalysts that may be advantageously employed in metathesis reactions are manufactured and sold by Materia, Inc. (Pasadena, California).

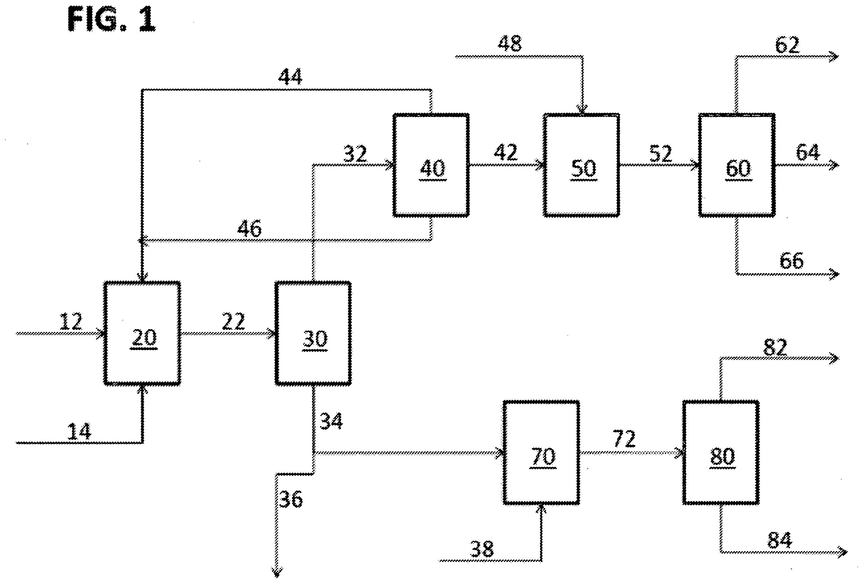

A process for metathesizing a natural oil and treating the resulting metathesized natural oil is illustrated in Figure 1. In the present disclosure, prior to the metathesis reaction, a natural oil feedstock may be treated to render the natural oil more suitable for the subsequent metathesis reaction. For example, the treatment of the natural oil involves the removal of catalyst poisons, such as peroxides, which may potentially diminish the activity of the metathesis catalyst. Non-limiting examples of natural oil feedstock treatment methods to diminish catalyst poisons include those described in PCT/US2008/09604, PCT/US2008/09635, and U.S. Patent Application Nos. 12/672,651 and 12/672,652. In certain examples of the present disclosure, the natural oil feedstock is thermally treated by heating the feedstock to a temperature greater than 100°C in the absence of oxygen and held at the temperature for a time sufficient to diminish catalyst poisons in the feedstock. In other examples, the temperature is between approximately 100°C and 300°C, between approximately 120°C and 250°C, between approximately 150°C and 210°C, or approximately between 190 and 200°C. In one example, the absence of oxygen is achieved by sparging the natural oil feedstock with nitrogen, wherein the nitrogen gas is pumped into the feedstock treatment vessel at a pressure of approximately 1034.21 kPa (10 atm)(150 psig).

In certain examples, the natural oil feedstock is chemically treated under conditions sufficient to diminish the catalyst poisons in the feedstock through a chemical reaction of the catalyst poisons. In certain examples, the feedstock is treated with a reducing agent or a cation-inorganic base composition. Non-limiting examples of reducing agents include bisulfate, borohydride, phosphine, thiosulfate, and combinations thereof.

In certain examples, the natural oil feedstock is treated with an adsorbent to remove catalyst poisons. In one example, the feedstock is treated with a combination of thermal and adsorbent methods. In another example, the feedstock is treated with a combination of chemical and adsorbent methods. In another example, the treatment involves a partial hydrogenation treatment to modify the natural oil feedstock's reactivity with the metathesis catalyst. Additional non-limiting examples of feedstock treatment are also described below when discussing the various metathesis catalysts.

Additionally, in certain examples, the low-molecular-weight olefin may also be treated prior to the metathesis reaction. Like the natural oil treatment, the low-molecular-weight olefin may be treated to remove poisons that may impact or diminish catalyst activity.

As shown in FIG. 1, after this optional treatment of the natural oil feedstock and/or low-molecular-weight olefin, the natural oil 12 is reacted with itself, or combined with a low-molecular-weight olefin 14 in a metathesis reactor 20 in the presence of a metathesis catalyst. In some embodiments, the natural oil 12 is soybean oil. Metathesis catalysts and metathesis reaction conditions are discussed in greater detail below. In certain embodiments, in the presence of a metathesis catalyst, the natural oil 12 undergoes a self-metathesis reaction with itself. In other embodiments, in the presence of the metathesis catalyst, the natural oil 12 undergoes a cross-metathesis reaction with the low-molecular-weight olefin 14. Also disclosed is that the natural oil 12 undergoes both self- and cross-metathesis reactions in parallel metathesis reactors. Multiple, parallel, or sequential metathesis reactions (at least one or more times) may be conducted. The self-metathesis and/or cross-metathesis reaction form a metathesized natural oil product 22 wherein the metathesized natural oil product 22 comprises olefins 32 and esters 34. In some embodiments, metathesized natural oil product 22 is metathesized soybean oil (MSBO). As used herein, "metathesized natural oil product" may also be referred to in the equivalent as "metathesized natural oil composition."

In the present disclosure, the low-molecular-weight olefin 14 is in the C₂ to C₆ range. As a non-limiting example, the low-molecular-weight olefin 14 may comprise at least one of the following: ethylene, propylene, 1-butene, 2-butene, isobutene, 1-pentene, 2-pentene, 3-pentene, 2-methyl-1-butene, 2-methyl-2-butene, 3-methyl-1-butene, cyclopentene, 1-hexene, 2-hexene, 3-hexene, 4-hexene, 2-methyl-1-pentene, 3-methyl-1-pentene, 4-methyl-1-pentene, 2-methyl-2-pentene, 3-methyl-2-pentene, 4-methyl-2-pentene, 2-methyl-3-pentene, and cyclohexene. In another example, the low-molecular-weight olefin 14 comprises at least one of styrene and vinyl cyclohexane. In another example, the low-molecular-weightolefin 14 may comprise at least one of ethylene, propylene, 1-butene, 2-butene, and isobutene. In another example, the low-molecular-weight olefin 14 comprises at least one alpha-olefin or terminal olefin in the C₂ to C₁₀ range.

In another example, the low-molecular-weight olefin 14 comprises at least one branched low-molecular-weight olefin in the C₄ to C₁₀ range. Non-limiting examples of branched low-molecular-weight olefins include isobutene, 3-methyl-1-butene, 2-methyl-3-pentene, and 2,2-dimethyl-3-pentene. By using these branched low-molecular-weight olefins in the metathesis reaction, the methathesized natural oil product will include branched olefins, which can be subsequently hydrogenated to iso-paraffins. In certain examples, the branched low-molecular-weight olefins may help achieve the desired performance properties for a fuel composition, such as jet, kerosene, or diesel fuel.

As noted, it is possible to use a mixture of various linear or branched low-molecular-weight olefins in the reaction to achieve the desired metathesis product distribution. In one example, a mixture of butenes (1-butene, 2-butenes, and, optionally, isobutene) may be employed as the low-molecular-weight olefin, offering a low cost, commercially available feedstock instead a purified source of one particular butene. Such low cost mixed butene feedstocks are typically diluted with n-butane and/or isobutane.

In the present disclosure, recycled streams from downstream separation units may be introduced to the metathesis reactor 20 in addition to the natural oil 12 and, in some examples, the low-molecular-weight olefin 14. For instance, in some examples, a C₂-C₆ recycle olefin stream or a C₃-C₄ bottoms stream from an overhead separation unit may be returned to the metathesis reactor. As shown in FIG. 1, a light weight olefin stream 44 from an olefin separation unit 40 may be returned to the metathesis reactor 20. In another example, the C₃-C₄ bottoms stream and the light weight olefin stream 44 are combined together and returned to the metathesis reactor 20. In another example, a C₁₅₊ bottoms stream 46 from the olefin separation unit 40 is returned to the metathesis reactor 20. In another example, all of the aforementioned recycle streams are returned to the metathesis reactor 20.

The metathesis reaction in the metathesis reactor 20 produces a metathesized natural oil product 22. In one example, the metathesized natural oil product 22 enters a flash vessel operated under temperature and pressure conditions which target C₂ or C₂-C₃ compounds to flash off and be removed overhead. The C₂ or C₂-C₃ light ends are comprised of a majority of hydrocarbon compounds having a carbon number of 2 or 3. In certain examples, the C₂ or C₂-C₃ light ends are then sent to an overhead separation unit, wherein the C₂ or C₂-C₃ compounds are further separated overhead from the heavier compounds that flashed off with the C₂-C₃ compounds. These heavier compounds are typically C₃-C₅ compounds carried overhead with the C₂ or C₂-C₃ compounds. After separation in the overhead separation unit, the overhead C₂ or C₂-C₃ stream may then be used as a fuel source. These hydrocarbons have their own value outside the scope of a fuel composition, and may be used or separated atthis stage for other valued compositions and applications. In certain examples, the bottoms stream from the overhead separation unit containing mostly C₃-C₅ compounds is returned as a recycle stream to the metathesis reactor. In the flash vessel, the metathesized natural oil product 22 that does not flash overhead is sent downstream for separation in a separation unit 30, such as a distillation column.

Prior to the separation unit 30, in certain examples, the metathesized natural oil product 22 may be introduced to an adsorbent bed to facilitate the separation of the metathesized natural oil product 22 from the metathesis catalyst. In one example, the adsorbent is a clay bed. The clay bed will adsorb the metathesis catalyst, and after a filtration step, the metathesized natural oil product 22 can be sent to the separation unit 30 for further processing. Separation unit 30 may comprise a distillation unit. In some embodiments, the distillation may be conducted, for example, by steam stripping the metathesized natural oil product. Distilling may be accomplished by sparging the mixture in a vessel, typically agitated, by contacting the mixture with a gaseous stream in a column that may contain typical distillation packing (e.g., random or structured), by vacuum distillation, or evaporating the lights in an evaporator such as a wiped film evaporator. Typically, steam stripping will be conducted at reduced pressure and at temperatures ranging from about 100° C. to 250° C. The temperature may depend, for example, on the level of vacuum used, with higher vacuum allowing for a lower temperature and allowing for a more efficient and complete separation of volatiles.

In another example, the adsorbent is a water soluble phosphine reagent such as tris hydroxymethyl phosphine (THMP). Catalyst may be separated with a water soluble phosphine through known liquid-liquid extraction mechanisms by decanting the aqueous phase from the organic phase. In other examples, the metathesized natural oil product 22 may be contacted with a reactant to deactivate or to extract the catalyst.

In the separation unit 30, in certain examples, the metathesized natural oil product 22 is separated into at least two product streams. In one embodiment, the metathesized natural oil product 22 is sent to the separation unit 30, or distillation column, to separate the olefins 32 from the esters 34. In another example, a byproduct stream comprising C₇'s and cyclohexadiene may be removed in a sidestream from the separation unit 30. In certain examples, the separated olefins 32 may comprise hydrocarbons with carbon numbers up to 24. In certain examples, the esters 34 may comprise metathesized glycerides. In other words, the lighter end olefins 32 are preferably separated or distilled overhead for processing into olefin compositions, while the esters 34, comprised mostly of compounds having carboxylic acid/ester functionality, are drawn into a bottoms stream. Based on the quality of the separation, it is possible for some ester compounds to be carried into the overhead olefin stream 32, and it is also possible for some heavier olefin hydrocarbons to be carried into the ester stream 34.

In the present disclosure, the olefins 32 may be collected and sold for any number of known uses. In other examples, the olefins 32 are further processed in an olefin separation unit 40 and/or hydrogenation unit 50 (where the olefinic bonds are saturated with hydrogen gas 48, as described below). In other examples, esters 34 comprising heavier end glycerides and free fatty acids are separated or distilled as a bottoms product for further processing into various products. In certain examples, further processing may target the production of the following non-limiting examples: fatty acid methyl esters; biodiesel; 9DA esters, 9UDA esters, and/or 9DDA esters; 9DA, 9UDA, and/or 9DDA; alkali metal salts and alkaline earth metal salts of 9DA, 9UDA, and/or 9DDA; diacids, and/or diesters of the transesterified products; and mixtures thereof. In certain examples, further processing may target the production of C₁₅-C₁₈ fatty acids and/or esters. In other examples, further processing may target the production of diacids and/or diesters. In yet other examples, further processing may target the production of compounds having molecular weights greater than the molecular weights of stearic acid and/or linolenic acid.

As shown in FIG. 1, regarding the overhead olefins 32 from the separation unit 30, the olefins 32 may be further separated or distilled in the olefin separation unit 40 to separate the stream's various components. In one example, light end olefins 44 consisting of mainly C₂-C₉ compounds may be distilled into an overhead stream from the olefin separation unit 40. In certain examples, the light end olefins 44 are comprised of a majority of C₃-C₈ hydrocarbon compounds. In other examples, heavier olefins having higher carbon numbers may be separated overhead into the light end olefin stream 44 to assist in targeting a specific fuel composition. The light end olefins 44 may be recycled to the metathesis reactor 20, purged from the system for further processing and sold, or a combination of the two. In one example, the light end olefins 44 may be partially purged from the system and partially recycled to the metathesis reactor 20. With regards to the other streams in the olefin separation unit 40, a heavier C₁₆₊, C₁₈₊, C₂₀₊, C₂₂₊, or C₂₄₊ compound stream may be separated out as an olefin bottoms stream 46. This olefin bottoms stream 46 may be purged or recycled to the metathesis reactor 20 for further processing, or a combination of the two. In another example, a center-cut olefin stream 42 may be separated out of the olefin distillation unit for further processing. The center-cut olefins 42 may be designed to target a selected carbon number range for a specific fuel composition. As a non-limiting example, a C₅-C₁₅ distribution may be targeted for further processing into a naphtha-type jet fuel. Alternatively, a C₃-C₁₆ distribution may be targeted for further processing into a kerosene-type jet fuel. In another example, a C₈-C₂₅ distribution may be targeted for further processing into a diesel fuel.

In certain examples, the olefins 32 may be oligomerized to form poly-alpha-olefins (PAOs) or poly-internal-olefins (PIOs), mineral oil substitutes, and/or biodiesel fuel. The oligomerization reaction may take place after the distillation unit 30 or after the overhead olefin separation unit 40. In certain examples, byproducts from the oligomerization reactions may be recycled back to the metathesis reactor 20 for further processing.

As mentioned, in one example, the olefins 32 from the separation unit 30 may be sent directly to the hydrogenation unit 50. In another example, the center-cut olefins 42 from the overhead olefin separation unit 40 may be sent to the hydrogenation unit 50. Hydrogenation may be conducted according to any known method in the art for hydrogenating double bond-containing compounds such as the olefins 32 or center-cut olefins 42. In certain examples, in the hydrogenation unit 50, hydrogen gas 48 is reacted with the olefins 32 or center-cut olefins 42 in the presence of a hydrogenation catalyst to produce a hydrogenated product 52.

In the present disclosure, the olefins are hydrogenated in the presence of a hydrogenation catalyst comprising nickel, copper, palladium, platinum, molybdenum, iron, ruthenium, osmium, rhodium, or iridium, individually or in combinations thereof. Useful catalyst may be heterogeneous or homogeneous. In some examples, the catalysts are supported nickel or sponge nickel type catalysts.

In some examples, the hydrogenation catalyst comprises nickel that has been chemically reduced with hydrogen to an active state (i.e., reduced nickel) provided on a support. The support may comprise porous silica (e.g., kieselguhr, infusorial, diatomaceous, or siliceous earth) or alumina. The catalysts are characterized by a high nickel surface area per gram of nickel.

Commercial examples of supported nickel hydrogenation catalysts include those available under the trade designations "NYSOFACT", "NYSOSEL", and "NI 5248 D" (from BASF Catalysts LLC, Iselin, NJ). Additional supported nickel hydrogenation catalysts include those commercially available under the trade designations "PRICAT 9910", "PRICAT 9920", "PRICAT 9908", "PRICAT 9936" (from Johnson Matthey Catalysts, Ward Hill, MA).

The supported nickel catalysts may be of the type described in U.S. Patent No. 3,351,566, US Patent No. 6,846,772, EP 0168091, and EP 0167201. Hydrogenation may be carried out in a batch or in a continuous process and may be partial hydrogenation or complete hydrogenation. In certain embodiments, the temperature ranges from about 50°C to about 350°C, about 100°C to about 300°C, about 150°C to about 250°C, or about 100°C to about 150°C. The desired temperature may vary, for example, with hydrogen gas pressure. Typically, a higher gas pressure will require a lower temperature. Hydrogen gas is pumped into the reaction vessel to achieve a desired pressure of H₂ gas. In certain examples, the H₂ gas pressure ranges from about 20684.27kPa (15 psig) (1 atm) to about 20684.27kPa (3000 psig) (204.1 atm), about 20684.27kPa (15 psig) (1 atm) to about 620.528kPa (90 psig) (6.1 atm), or about 689.476kPa (100 psig) (6.8 atm) to about 3447.38kPa (500 psig) (34 atm). In certain examples, the reaction conditions are "mild," wherein the temperature is approximately between approximately 50°C and approximately 100°C and the H₂ gas pressure is less than approximately 689.476kPa (100 psig). In other examples, the temperature is between about 100°C and about 150°C, and the pressure is between about 689.476kPa (100 psig) (6.8 atm) and about 3447.38 (500 psig) (34 atm). When the desired degree of hydrogenation is reached, the reaction mass is cooled to the desired filtration temperature.

During hydrogenation, the carbon-carbon double bond containing compounds in the olefins are partially to fully saturated by the hydrogen gas 48. In one example, the resulting hydrogenated product 52 includes hydrocarbons with a distribution centered between approximately C₁₀ and C₁₂ hydrocarbons for naphtha- and kerosene-type jet fuel compositions. In another example, the distribution is centered between approximately C₁₆ and C₁₈ for a diesel fuel composition.

In certain examples, based upon the quality of the hydrogenated product 52 produced in the hydrogenation unit 50, it may be preferable to isomerize the olefin hydrogenated product 52 to assist in targeting of desired fuel properties such as flash point, freeze point, energy density, cetane number, or end point distillation temperature, among other parameters. Isomerization reactions are well-known in the art, as described in U.S. Patent Nos. 3,150,205; 4,210,771; 5,095,169; and 6,214,764. In one example, the isomerization reaction at this stage may also crack some of the C₁₅₊ compounds remaining, which may further assist in producing a fuel composition having compounds within the desired carbon number range, such as 5 to 16 for a jet fuel composition.

In certain examples, the isomerization may occur concurrently with the hydrogenation step in the hydrogenation unit 50, thereby targeting a desired fuel product. In other examples, the isomerization step may occur before the hydrogenation step (i.e., the olefins 32 or center-cut olefins 42 may be isomerized before the hydrogenation unit 50). In yet other example, it is possible that the isomerization step may be avoided or reduced in scope based upon the selection of low-molecular-weight olefin(s) 14 used in the metathesis reaction.

In the present disclosure, the hydrogenated product 52 comprises approximately 15-25 weight % C₇, approximately <5 weight % C₈, approximately 20-40 weight % Cg, approximately 20-40 weight % C₁₀, approximately <5 weight % C₁₁, approximately 15-25weight % C₁₂, approximately <5 weight % C₁₃, approximately <5 weight % C₁₄, approximately <5 weight % C₁₅, approximately <1 weight % C₁₆, approximately <1 weight % C₁₇, and approximately <1 weight % C₁₈+. In certain embodiments, the hydrogenated product 52 comprises a heat of combustion of at least approximately 40, 41, 42, 43 or 44 MJ/kg (as measured by ASTM D3338). In certain examples, the hydrogenated product 52 contains less than approximately 1 mg sulfur per kg hydrogenated product (as measured by ASTM D5453). In other examples, the hydrogenated product 52 comprises a density of approximately 0.70-0.75 (as measured by ASTM D4052). In other examples, the hydrogenated product has a final boiling point of approximately 220-240°C (as measured by ASTM D86).

The hydrogenated product 52 produced from the hydrogenation unit 50 may be used as a fuel composition, non-limiting examples of which include jet, kerosene, or diesel fuel. In certain examples, the hydrogenated product 52 may contain byproducts from the hydrogenation, isomerization, and/or metathesis reactions. As shown in FIG. 1, the hydrogenated product 52 may be further processed in a fuel composition separation unit 60, removing any remaining byproducts from the hydrogenated product 52, such as hydrogen gas, water, C₂-C₉ hydrocarbons, or C₁₅+ hydrocarbons, thereby producing a targeted fuel composition. In one example, the hydrogenated product 52 may be separated into the desired fuel C₉-C₁₅ product 64, and a light-ends C₂-C₉ fraction 62 and/or a C₁₅+ heavy-ends fraction 66. Distillation may be used to separate the fractions. Alternatively, in other examples, such as for a naphtha- or kerosene-type jet fuel composition, the heavy ends fraction 66 can be separated from the desired fuel product 64 by cooling the hydrogenated product 52 to approximately -40°C, -47°C, or -65°C and then removing the solid, heavy ends fraction 66 by techniques known in the art such as filtration, decantation, or centrifugation.

With regard to the esters 34 from the distillation unit 30, in certain examples, the esters 34 may be entirely withdrawn as an ester product stream 36 and processed further or sold for its own value, as shown in FIG. 1. As a non-limiting example, the esters 34 may comprise various triglycerides that could be used as a lubricant. Based upon the quality of separation between olefins and esters, the esters 34 may comprise some heavier olefin components carried with the triglycerides. In other examples, the esters 34 may be further processed in a biorefinery or another chemical or fuel processing unit known in the art, thereby producing various products such as biodiesel or specialty chemicals that have higher value than that of the triglycerides, for example. Alternatively, in certain embodiments, the esters 34 may be partially withdrawn from the system and sold, with the remainder further processed in the biorefinery or another chemical or fuel processing unit known in the art.

In certain embodiments, the ester stream 34 is sent to a transesterification unit 70. Within the transesterification unit 70, the esters 34 are reacted with at least one alcohol 38 in the presence of a transesterification catalyst. In certain embodiments, the alcohol comprises methanol and/or ethanol. In one example, the transesterification reaction is conducted at approximately 60-70°C and approximately 1 atm. In certain examples, the transesterification catalyst is a homogeneous sodium methoxide catalyst. Varying amounts of catalyst may be used in the reaction, and, in certain examples, the transesterification catalyst is present in the amount of approximately 0.5-1.0 weight % of the esters 34.

The transesterification reaction may produce transesterified products 72 including saturated and/or unsaturated fatty acid methyl esters ("FAME"), glycerin, methanol, and/or free fatty acids. In certain examples, the transesterified products 72, or a fraction thereof, may comprise a source for biodiesel. In certain examples, the transesterified products 72 comprise 9DA esters, 9UDA esters, and/or 9DDA esters. Non-limiting examples of 9DA esters, 9UDA esters and 9DDA esters include methyl 9-decenoate ("9-DAME"), methyl 9-undecenoate ("9-UDAME"), and methyl 9-dodecenoate ("9-DDAME"), respectively. As a non-limiting example, in a transesterification reaction, a 9DA moiety of a metathesized glyceride is removed from the glycerol backbone to form a 9DA ester.

In another example, a glycerin alcohol may be used in the reaction with a glyceride stream. This reaction may produce monoglycerides and/or diglycerides. In certain examples, the transesterified products 72 from the transesterification unit 70 can be sent to a liquid-liquid separation unit, wherein the transesterified products 72 (i.e., FAME, free fatty acids, and/or alcohols) are separated from glycerin. Additionally, in certain examples, the glycerin byproduct stream may be further processed in a secondary separation unit, wherein the glycerin is removed and any remaining alcohols are recycled back to the transesterification unit 70 for further processing.

In one example, the transesterified products 72 are further processed in a water-washing unit. In this unit, the transesterified products undergo a liquid-liquid extraction when washed with water. Excess alcohol, water, and glycerin are removed from the transesterified products 72. In another example, the water-washing step is followed by a drying unit in which excess water is further removed from the desired mixture of esters (*i.e.*, specialty chemicals). Such specialty chemicals include non-limiting examples such as 9DA, 9UDA, and/or 9DDA, alkali metal salts and alkaline earth metal salts of the preceding, individually or in combinations thereof.

In one example, the specialty chemical (e.g., 9DA) may be further processed in an oligomerization reaction to form a lactone, which may serve as a precursor to a surfactant.

In certain examples, the transesterifed products 72 from the transesterification unit 70 or specialty chemicals from the water-washing unit or drying unit are sent to an ester distillation column 80 for further separation of various individual or groups of compounds, as shown in FIG. 1. This separation may include, but is not limited to, the separation of 9DA esters, 9UDA esters, and/or 9DDA esters. In one example, the 9DA ester 82 may be distilled or individually separated from the remaining mixture 84 of transesterified products or specialty chemicals. In certain process conditions, the 9DA ester 82 should be the lightest component in the transesterified product or specialty chemical stream, and come out at the top of the ester distillation column 80. In another example, the remaining mixture 84, or heavier components, of the transesterified products or specialty chemicals may be separated off the bottom end of the column. In certain examples, this bottoms stream 84 may potentially be sold as biodiesel.

The 9DA esters, 9UDA esters, and/or 9DDA esters may be further processed after the distillation step in the ester distillation column. In one example, under known operating conditions, the 9DA ester, 9UDA ester, and/or 9DDA ester may then undergo a hydrolysis reaction with water to form 9DA, 9UDA, and/or 9DDA, alkali metal salts and alkaline earth metal salts of the preceding, individually or in combinations thereof.

In certain examples, the fatty acid methyl esters from the transesterified products 72 may be reacted with each other to form other specialty chemicals such as dimers.

Multiple, sequential metathesis reaction steps may be employed. For example, the metathesized natural oil product may be made by reacting a natural oil in the presence of a metathesis catalyst to form a first metathesized natural oil product. The first metathesized natural oil product may then be reacted in a self-metathesis reaction to form another metathesized natural oil product. Alternatively, the first metathesized natural oil product may be reacted in a cross-metathesis reaction with a natural oil to form another metathesized natural oil product. Also in the alternative, the transesterified products, the olefins and/or esters may be further metathesized in the presence of a metathesis catalyst. Such multiple and/or sequential metathesis reactions can be performed as many times as needed, and at least one or more times, depending on the processing/compositional requirements as understood by a person skilled in the art. In some embodiments of a multiple metathesis reaction, the second or subsequent metathesis will produce a distinguishable product if the composition is altered. For example, if soybean oil is cross metathesized with a low weight olefin, such as 1-butene, in a first metathesis, it will produce a higher molecular weight oligomeric mixture upon a second metathesis if the olefins that were present during the first metathesis have been partially removed prior to or during the second metathesis. This generally is done by stripping the olefins, in this instance from the butenolyzed soybean oil. This "compositional shift" allows further reaction to occur during the second metathesis. Absent such a shift, adding more catalyst to a system at equilibrium will result in little or no change.

As used herein, a "metathesized natural oil product" may include products that have been once metathesized and/or multiply metathesized. These procedures may be used to form metathesis dimers, metathesis trimers, metathesis tetramers, metathesis pentamers, and higher order metathesis oligomers (e.g., metathesis hexamers, metathesis heptamers, metathesis octamers, metathesis nonamers, metathesis decamers, and higher than metathesis decamers). These procedures can be repeated as many times as desired (for example, from 2 to about 50 times, or from 2 to about 30 times, or from 2 to about 10 times, or from 2 to about 5 times, or from 2 to about 4 times, or 2 or 3 times) to provide the desired metathesis oligomer or polymer which may comprise, for example, from 2 to about 100 bonded groups, or from 2 to about 50, or from 2 to about 30, or from 2 to about 10, or from 2 to about 8, or from 2 to about 6 bonded groups, or from 2 to about 4 bonded groups, or from 2 to about 3 bonded groups. In the present disclosure, it may be desirable to use the metathesized natural products produced by cross metathesis of a natural oil, or blend of natural oils, with a C2-C100 olefin, as the reactant in a self metathesis reaction to produce another metathesized natural oil product. Alternatively, metathesized natural products produced by cross metathesis of a natural oil, or blend of natural oils, with a C2-C100 olefin can be combined with a natural oil, or blend of natural oils, and further metathesized to produce another metathesized natural oil product.

The metathesized natural oil product may have a number average molecular weight in the range from about 100 g/mol to about 150,000 g/mol, or from about 300 g/mol to about 100,000 g/mol, or from about 300 g/mol to about 70,000 g/mol, or from about 300 g/mol to about 50,000 g/mol, or from about 500 g/mol to about 30,000 g/mol, or from about 700 g/mol to about 10,000 g/mol, or from about 1,000 g/mol to about 5,000 g/mol, but according to the present invention is from 2,000 g/mol to 4,000 g/mol. The metathesized natural oil product may have a weight average molecular weight in the range from about from about 1,000 g/mol to about 100,000 g/mol, from about 2,500 g/mol to about 50,000 g/mol, from about 4,000 g/mol to about 30,000 g/mol, from about 5,000 g/mol to about 20,000 g/mol, and from about 6,000 g/mol to about 15,000 g/mol, but according to the present invention is from 7,000 g/mol to 35,000 g/mol. The metathesized natural oil product may have a z-average molecular weight in the range from about from about 5,000 g/mol to about 1,000,000 g/mol, for example from about 7,500 g/mol to about 500,000 g/mol, from about 10,000 g/mol to about 300,000 g/mol, or from about 12,500 g/mol to about 200,000 g/mol, but according to the present invention is from 10,000 g/mol to 125,000 g/mol. The polydispersity index is calculated by dividing the weight average molecular weight by the number average molecular weight. Polydispersity is a measure of the breadth of the molecular weight distribution of the metathesized natural oil product, and such products generally exhibit a polydispersity index of about 1 to about 20, or from about 2 to about 15, but according to the present invention of from 2 to 12. The number average molecular weight, weight average molecular weight, and z-average molecularweight may be determined by gel permeation chromatography (GPC), gas chromatography, gas chromatography mass-spectroscopy, NMR spectroscopy, vapor phase osmometry (VPO), wet analytical techniques such as acid number, base number, saponification number or oxirane number, and the like. In the present diclosure, gas chromatography and gas chromatography mass-spectroscopy can be used to analyze the metathesized natural oil product by first transforming the triglycerides to their corresponding methyl esters prior to testing. The extent to which the individual triglyceride molecules have been polymerized can be understood as being directly related to the concentration of diester molecules found in the analyzed fatty acid methyl esters. In some embodiments, the molecular weight of the metathesized natural oil product can be increased by transesterifying the metathesized natural oil product with diesters. In some embodiments, the molecular weight of the metathesized natural oil product can be increased by esterifying the metathesized natural oil product with diacids. In the present disclosure, the metathesized natural oil product has a dynamic viscosity between about 0.001 Pa·s (1 centipoise (cP)) and about 10 Pa·s (10,000 centipoise (cP)), between about 0.3 Pa·s (30 centipoise (cP)) and about 5 Pa·s (5000 cP), between about 0.05 Pa·s (50 cP) and about 3 Pa·s (3000 cP), and from between about 0.8 Pa·s (80 cP) and about 1.5 Pa·s (1500 cP), but according to the present invention has a dynamic viscosity at 40°C of between 0.2 Pa·s (200 cP) and 3.5 Pa·s (3500 cP), and a dynamic viscosity at 100°C of between 0.075 Pa·s (75 cP) and 0.6 Pa·s (600 cP).

The metathesis process can be conducted under any conditions adequate to produce the desired metathesis products. For example, stoichiometry, atmosphere, solvent, temperature, and pressure can be selected by one skilled in the art to produce a desired product and to minimize undesirable byproducts. The metathesis process may be conducted under an inert atmosphere. Similarly, if a reagent is supplied as a gas, an inert gaseous diluent can be used. The inert atmosphere or inert gaseous diluent typically is an inert gas, meaning that the gas does not interact with the metathesis catalyst to substantially impede catalysis. For example, particular inert gases are selected from the group consisting of helium, neon, argon, nitrogen, individually or in combinations thereof.

In certain examples, the metathesis catalyst is dissolved in a solvent prior to conducting the metathesis reaction. In certain examples, the solvent chosen may be selected to be substantially inert with respect to the metathesis catalyst. For example, substantially inert solvents include, without limitation, aromatic hydrocarbons, such as benzene, toluene, xylenes, etc.; halogenated aromatic hydrocarbons, such as chlorobenzene and dichlorobenzene; aliphatic solvents, including pentane, hexane, heptane, cyclohexane, etc.; and chlorinated alkanes, such as dichloromethane, chloroform, dichloroethane, etc. In one particular embodiment, the solvent comprises toluene. The metathesis reaction temperature may be a rate-controlling variable where the temperature is selected to provide a desired product at an acceptable rate. In the present disclosure, the metathesis reaction temperature is greater than about - 40°C, greater than about -20°C, greater than about 0°C, or greater than about 10°C. In certain examples, the metathesis reaction temperature is less than about 150°C, or less than about 120°C. In one example, the metathesis reaction temperature is between about 10°C and about 120°C.

The metathesis reaction can be run under any desired pressure. Typically, it will be desirable to maintain a total pressure that is high enough to keep the cross-metathesis reagent in solution. Therefore, as the molecular weight of the cross- metathesis reagent increases, the lower pressure range typically decreases since the boiling point of the cross-metathesis reagent increases. The total pressure may be selected to be greater than about 10 kPa (0.1 atm), in some embodiments greater than about 30 kPa (0.3 atm), or greater than about 100 kPa (1 atm). Typically, the reaction pressure is no more than about 7000 kPa (70 atm), in some embodiments no more than about 3000 kPa 30 atm. A non-limiting exemplary pressure range for the metathesis reaction is from about 100kPa (1 atm) to about 3000 kPa (30 atm). In certain embodiments it may be desirable to run the metathesis reactions under an atmosphere of reduced pressure. Conditions of reduced pressure or vacuum may be used to remove olefins as they are generated in a metathesis reaction, thereby driving the metathesis equilibrium towards the formation of less volatile products. In the case of a self-metathesis of a natural oil, reduced pressure can be used to remove C12 or lighter olefins including, but not limited to, hexene, nonene, and dodecene, as well as byproducts including, but not limited to cyclohexadiene and benzene as the metathesis reaction proceeds. The removal of these species can be used as a means to drive the reaction towards the formation of diester groups and cross linked triglycerides.

The metathesized natural oil compositions described herein may be utilized independently and/or incorporated into various formulations and used as functional ingredients in dimethicone replacements, laundry detergents, fabric softeners, personal care applications, such as emollients, hair fixative polymers, rheology modifiers, specialty conditioning polymers, surfactants, UV absorbers, solvents, humectants, occlusives, film formers, or as end use personal care applications, such as cosmetics, lip balms, lipsticks, hairdressings, sun care products, moisturizer, fragrance sticks, perfume carriers, skin feel agents, shampoos/conditioners, bar soaps, hand soaps/washes, bubble baths, body washes, facial cleansers, shower gels, wipes, baby cleansing products, creams/lotions, and antiperspirants/deodorants.

The metathesized natural oil compositions described herein may also be incorporated into various formulations and used as functional ingredients in lubricants, functional fluids, fuels and fuel additives, additives for such lubricants, functional fluids and fuels, plasticizers, asphalt additives, friction reducing agents, antistatic agents in the textile and plastics industries, flotation agents, gelling agents, epoxy curing agents, corrosion inhibitors, pigment wetting agents, in cleaning compositions, plastics, coatings, adhesives, skin feel agents, film formers, rheological modifiers, release agents, conditioners dispersants, hydrotropes, industrial and institutional cleaning products, oilfield applications, gypsum foamers, sealants, agricultural formulations, enhanced oil recovery compositions, solvent products, gypsum products, gels, semi-solids, detergents, heavy duty liquid detergents (HDL), light duty liquid detergents (LDL), liquid detergent softeners, antistat formulations, dryer softeners, hard surface cleaners (HSC) for household, autodishes, rinse aids, laundry additives, carpet cleaners, softergents, single rinse fabric softeners, I&I laundry, oven cleaners, car washes, transportation cleaners, drain cleaners, defoamers, anti-foamers, foam boosters, anti-dust/dust repellants, industrial cleaners, institutional cleaners, janitorial cleaners, glass cleaners, graffiti removers, concrete cleaners, metal/machine parts cleaners, pesticides, agricultural formulations and food service cleaners, plasticizers, asphalt additives and emulsifiers, friction reducing agents, film formers, rheological modifiers, biocides, biocide potentiators, release agents, household cleaning products, including liquid and powdered laundry detergents, liquid and sheet fabric softeners, hard and soft surface cleaners, sanitizers and disinfectants, and industrial cleaning products, emulsion polymerization, including processes for the manufacture of latex and for use as surfactants as wetting agents, and in agriculture applications as formulation inerts in pesticide applications or as adjuvants used in conjunction with the delivery of pesticides including agricultural crop protection turf and ornamental, home and garden, and professional applications, and institutional cleaning products, oil field applications, including oil and gas transport, production, stimulation and drilling chemicals and reservoir conformance and enhancement, organoclays for drilling muds, specialty foamers for foam control or dispersancy in the manufacturing process of gypsum, cement wall board, concrete additives and firefighting foams, paints and coalescing agents, paint thickeners, or other applications requiring cold tolerance performance or winterization (e.g., applications requiring cold weather performance without the inclusion of additional volatile components).

### EXAMPLES

### Multiple Metathesis Example

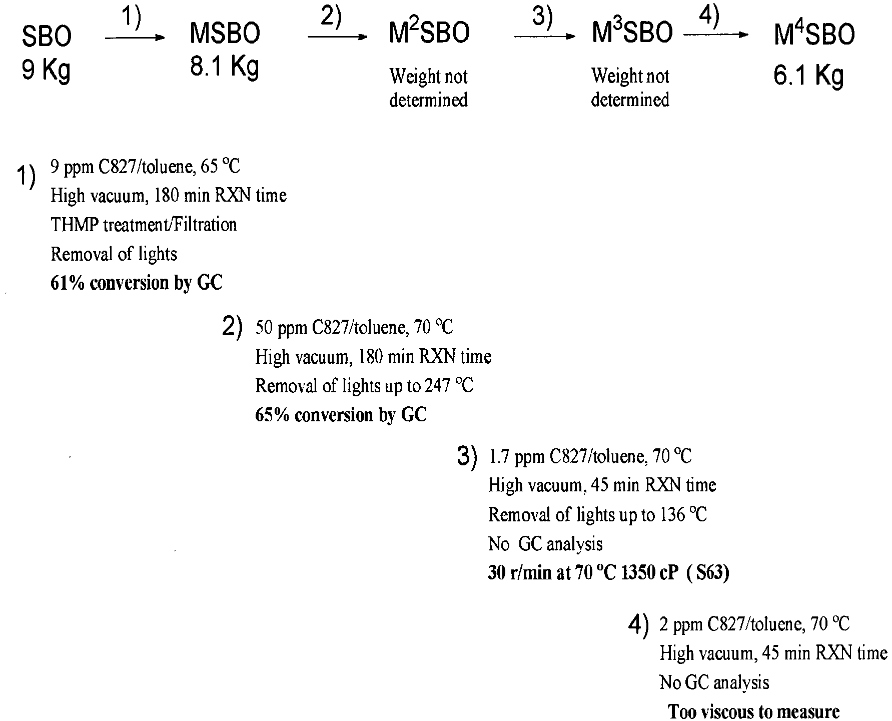

### Physical Properties of Metathesized Soybean Oil (Samples A. B and C)

**SAMPLE A**

| Kinematic Viscosity @ 40 °C (ASTM D445, in cm²/s (cSt)) | KinematicViscosity @ 100°C (ASTM D445, in cm²/s (cSt)) | Viscosity Index | Noack Volatility (ASTM D5800) | Pour Point (ASTM D97, in °C) |
|---|---|---|---|---|
| 2.46 (246cSt) | 0.4 (40cSt) | 219 | 8.3 wt % | 6 |

**SAMPLE B**

| Brookfield Viscosity Pa·s (cP) | Flash Point (ASTM D93, in °C) | Pour Point (ASTM D97, in °C) |
|---|---|---|
| 0.295 Pa·s (295cP) | 177 | 3 |

**SAMPLE C**

| | | |
|---|---|---|
| Once metathesized (1x) | unstripped | 0.135 Pa·s (135 cP) |
| Once metathesized (1x) | stripped at 150°C | 0.2625 Pa·s (262.5 cP) |
| Once metathesized (1x) | stripped at 200°C | 0.305 Pa·s (305 cP) |
| Twice metathesized (2x) | unstripped | 0.3925 Pa·s (392.5 cP) |
| Twice metathesized (2x) | stripped at 150°C | 0.5675 Pa·s (567.5 cP) |
| Twice metathesized (2x) | stripped at 200°C | 0.65 Pa·s (650 cP) |

Viscosity measurements for Sample C were performed at 40°C.

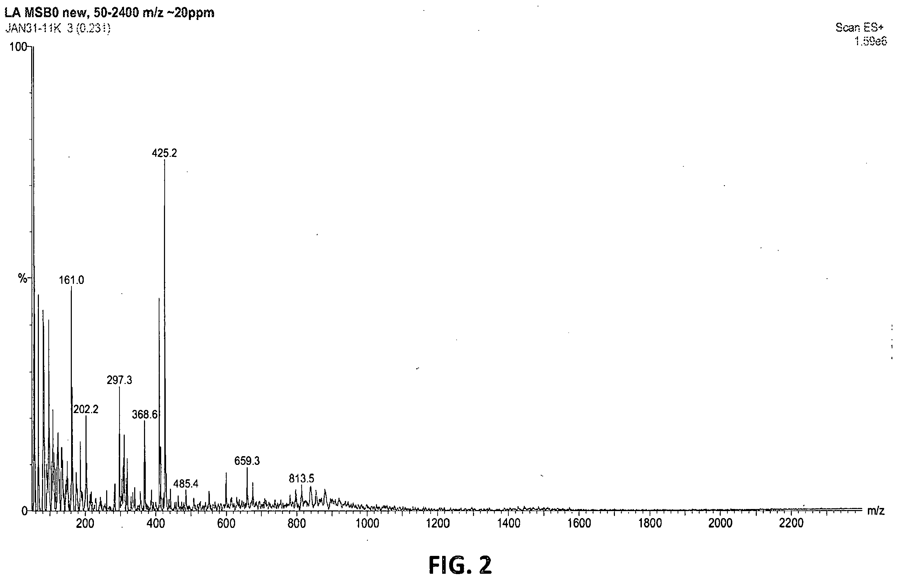

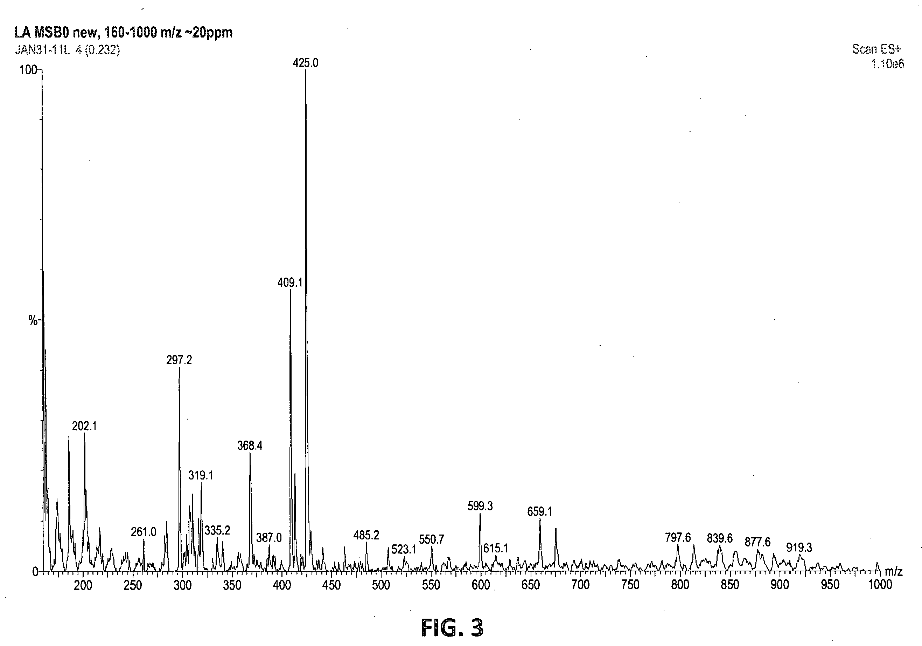

### Various compositional fractions of MSBO. Reference FIG. 2 and FIG. 3 for mass spectra analyses.

### 1. peak at 368

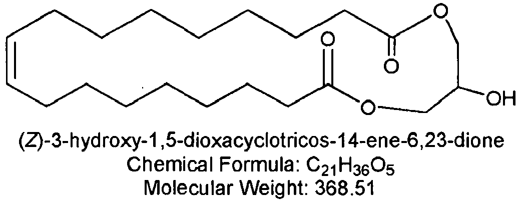

### 2. peak at 409

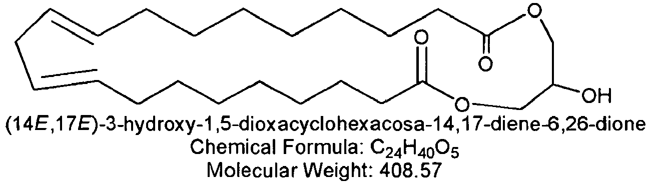

### 3. Peak at 659

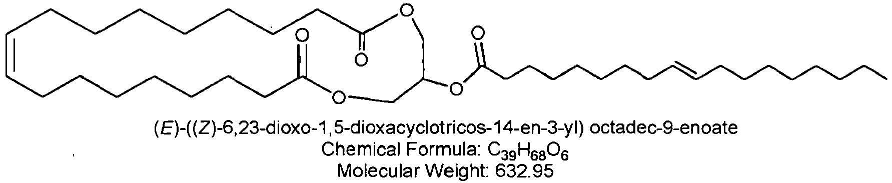

M+Na (23)= 655.95
M+ K(39)=671.95

### 4. peak at 675

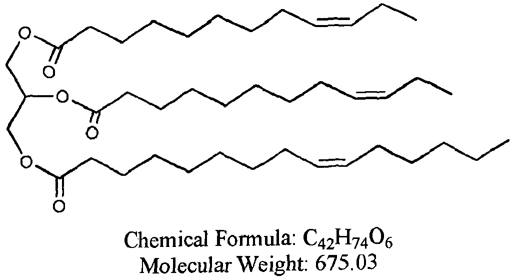

### 5. peak at 797

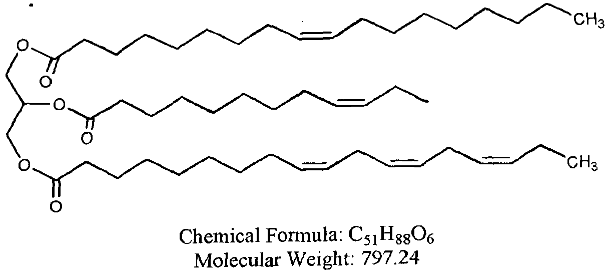

### 6. peak at 877.6

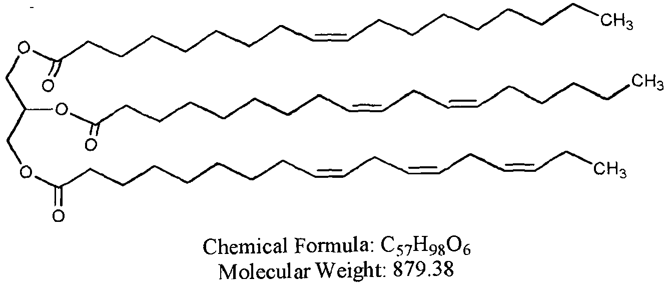

### 7. peak ∼919

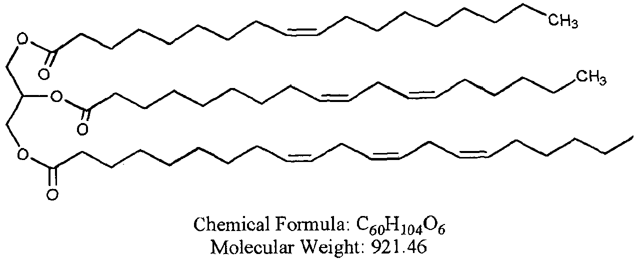

### Metathesis reaction of Triolein (oleyl triglyceride) with Grubbs 2^{nd} generation catalyst

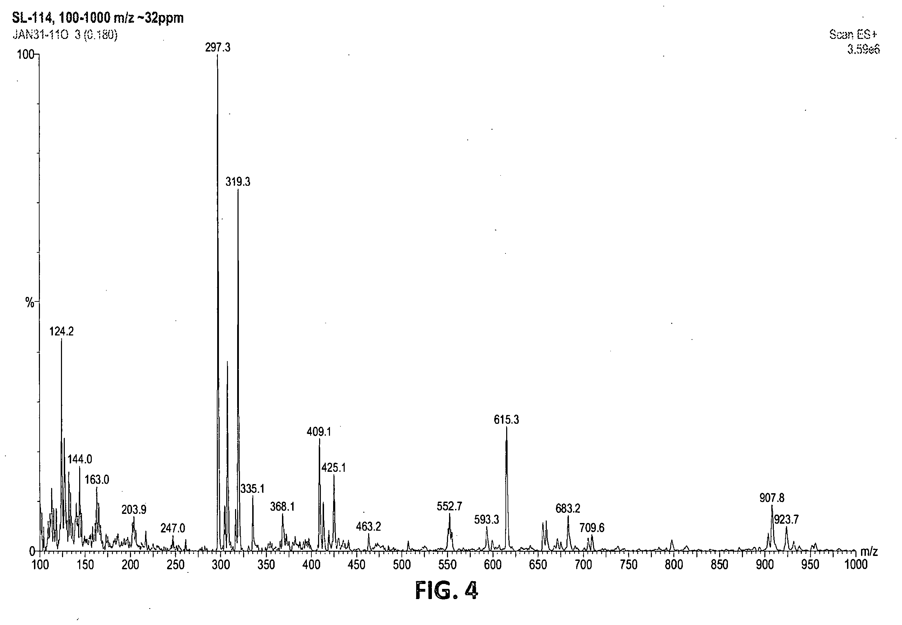

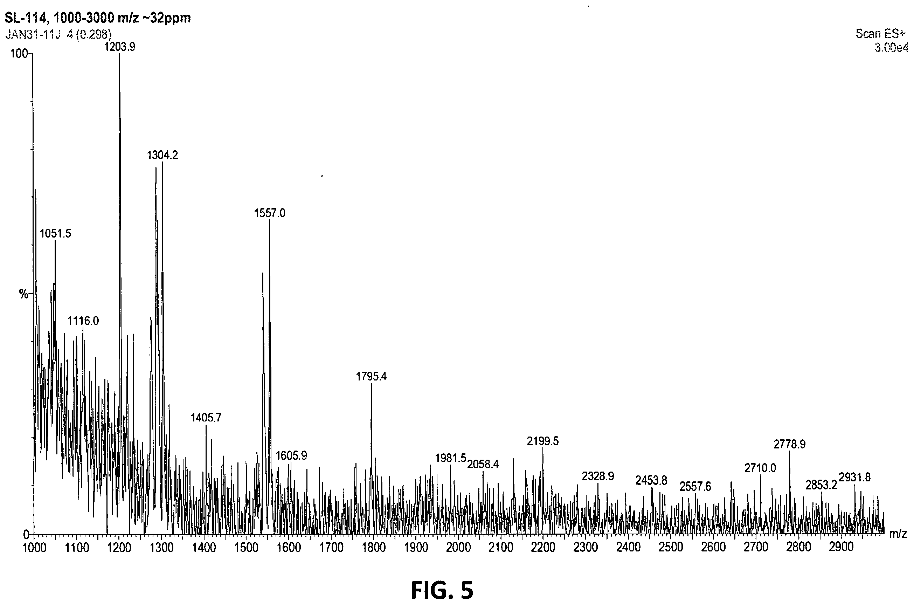

1 gram Triolein in a flask was heated to 45°C under N₂ protection. 0.01 gram Catalyst was added. They reaction was kept at 45°C for 16 hours and quenched with ethyl vinyl ether. The mixture was dissolved in Ethyl acetate and filtered through celite. The MS of resultant samples was tested on a triple quadrupole mass spectrometer with electrospray ionization source for Micromass Quattro LC. Reference FIG. 4 and FIG. 5 for mass spectra analyses.

### 1. peak at 907

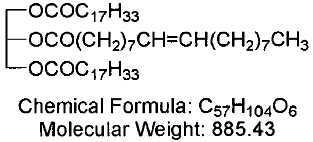

M+Na (23)= 908
M+ K(39)=924

### 2. peak at 409

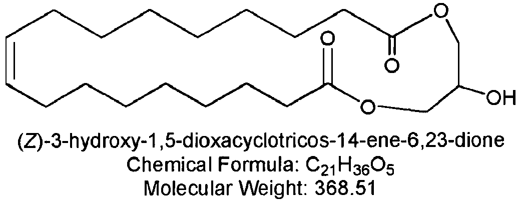

M+Na (23)= 391.51
M+ K(39)=407.51

### 3. Peak at ∼655

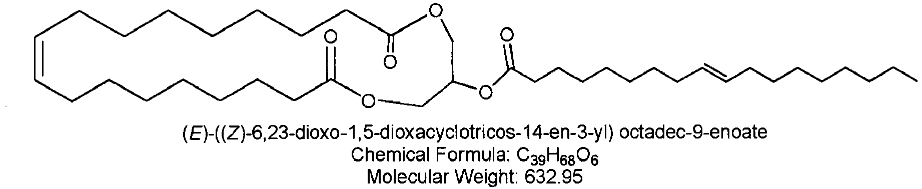

M+Na (23)= 655.95
M+ K(39)=671.95

### 4. Peak at 1051

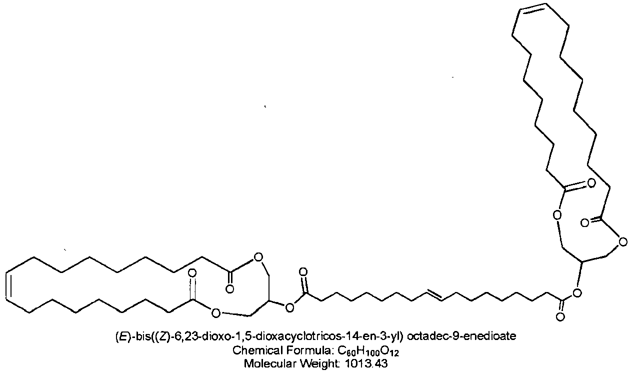

M+Na (23)= 1036
M+ K(39)= 1052

### 5. Peak at 1557

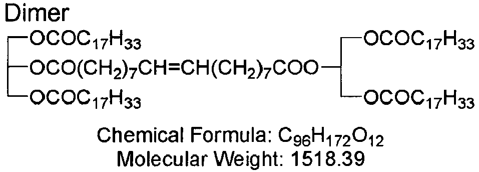

M+ Na(23)=1541
M+ K(39) =1557

### 6. Peak at 2199

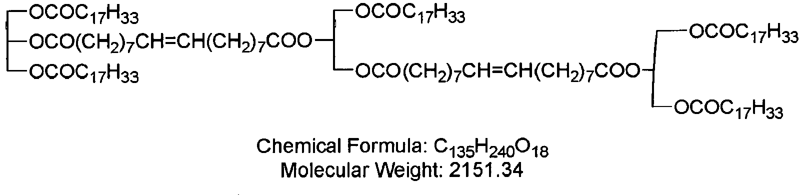

M+Na (23)=2174
M+ K(39)=2190

### 7. Peak at -1300

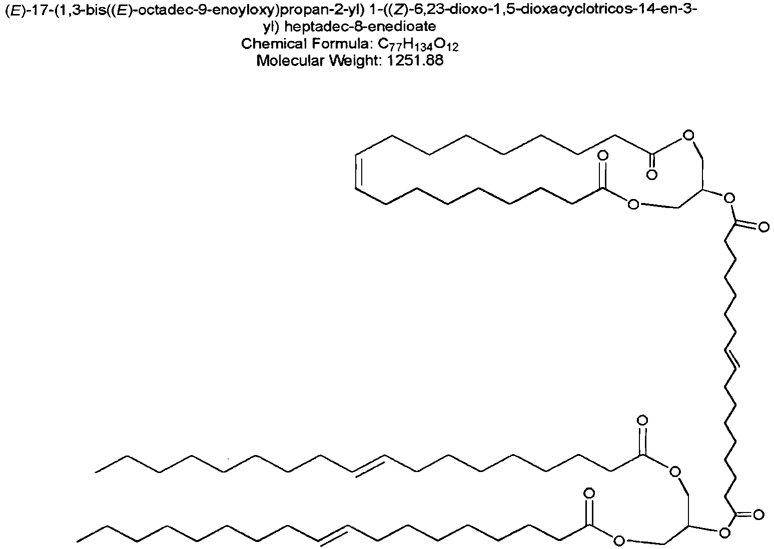

M+Na (23)= 1274
M+ K(39)=1290.88

### Metathesis of Canola Oil to Generate FAME and diesters

Refined bleached and deodorized canola oil (170 g) was loaded into a 250 ml 2-neck round bottom flask with a magnetic stir bar. The top joint of the flask was fitted with a 320 mm cold coil condenser fed by a chiller circulator set to 15° C. To the top of the condenser was fitted a hose adapter connected to an oil bubbler via Tygon tubing. The side arm neck was fitted with a rubber septum through which was fed a needle type thermocouple and an 18 gauge stainless steel needle for the purpose of supplying the flask with a nitrogen source. The oil was heated, with magnetic stirring, for 2 hr at 200° C while being sparged with dry nitrogen. After 2 hr, the oil was allowed to cool to 70° C, before adding the metathesis catalyst. The catalyst (Materia C827) was added via canola oil slurry through the top joint of the flask with a nitrogen sweep being maintained throughout the slurry addition. At this time the coil condenser was replaced. The reaction mixture was sparged with nitrogen an additional five minutes, to insure an inert atmosphere, before the nitrogen supply was closed. Metathesis was carried out for 3 hr at 70° C, with no nitrogen sweep, before raising the temperature of the reaction mixture to 100° C for the purpose of deactivating the catalyst. Following 1 hr at 100° C, the reaction mixture was allowed to cool to ambient temperature overnight, under a slow nitrogen sweep.

The next day, the rubber septum was exchanged for PTFE thermocouple adapter, and the coil condenser was exchanged for a short path distillation head with jacketed condenser equipped with a 100 ml receiving flask. The reaction mixture was stripped to a pot temperature of 250° C at a pressure of 39.997Pa (300 mTorr) for 4.5 hr. Stripping resulted in the removal of 17.4 wt. % lights and yielded a slightly burnt looking oil product. Brookfield viscosity of the final product was measured as 0.71 Pa·s (710 cp) at 40° C. Conversion of the product to its corresponding fatty acid methyl esters was accomplished prior to analysis by gas chromatography. The resulting mixture of fatty acid methyl esters was found to contain 27 wt. % diesters as shown by gas chromatography.

### Data Set #1 on Metathesized Natural Oil Compositions

In the Data Sets below, Mn refers to number average molecular weight, Mw refers to weight average molecular weight, Mz refers to z average molecular weight, MP refers to peak molecular weight, PDI refers to polydispersity index, TGA refers to thermogravimetric analysis, and IV refers to intrinsic viscosity. Also, MSBO refers to metathesized soybean oil, MCO refers to metathesized canola oil, and 2X refers to twice metathesized.

| Notebook # | Product | Description | Diester Content (%) | Mn | Mw | Mz | PDI | Dynamic Viscosity @ 40C | TGA | IV |
|---|---|---|---|---|---|---|---|---|---|---|
| 1033-96-5 | MSBO | Stripped, filtered | 16.85 | 2461 | 6946 | 12987 | 2.82 | 0.281 Pa·s (281 cP) | n/a | n/a |
| 1064-6-4 | 2XMSBO | 2XMSBO Stripped at 200°C | 22.17 | 3034 | 12827 | 38689 | 4.23 | 0.65Pa·s (650cP) | n/a | 108.54 |
| 1106-94-C | MCO | Super gel | 31.72 | 2653 | 27098 | 121316 | 10.21 | Too Viscous to measure | n/a | n/a |
| 1106-96-D | MCO | n/a | n/a | 2982 | 11142 | 32490 | 3.74 | 0.7Pa·s (700cP) | n/a | 77.52 |
| 1106-98-D | MCO | n/a | n/a | 2844 | 10603 | 29450 | 3.73 | 0.75Pa·s (750cP) | n/a | 91.17 |
| 1129-12-2 | 2X MSBO | Stripped at 200°C | 25.06 | 2336 | 9693 | 28181 | 4.15 | 0.642Pa·s (642cP) | 2.37% | 85.62 |
| 1129-13-1 | MSBO | Stripped at 200°C | 24.26 | 2363 | 7363 | 18648 | 3.11 | 0.474Pa·s (474cP) | n/a | n/a |
| 1129-13-2 | 2X MSBO | Stripped at 200°C | n/a | n/a | n/a | n/a | n/a | n/a | n/a | n/a |
| BB9004 | MSBO | Stripped at 150°C | 26.73 | 3393 | 20608 | 63294 | 6.07 | 1.525Pa·s (1525cP) | n/a | n/a |
| BB9012 | MSBO | Stripped at 150°C | 23.48 | 2933 | 15175 | 44975 | 5.17 | 0.990Pa·s 990cP | n/a | n/a |
| BB9013 | MSBO | Stripped at 150°C | 23.29 | 3007 | 15899 | 47980 | 5.29 | 1.215Pa·s (1215cP) | n/a | n/a |
| BB9014 | 2XMSBO | 2XMSBO Stripped at 150°C | 23.17 | 2779 | 13302 | 45017 | 4.79 | 0.590Pa·s (590cP) | n/a | 112.50 |
| BB9015 | 2XMSBO | 2XMSBO Stripped at 200°C | 23.03 | 2691 | 12142 | 41782 | 4.51 | 0.635Pa·s (635cP) | n/a | 113.88 |

### Data Set #2 on Metathesized Natural Oil Compositions

| | **Sample no.** | **Feedstock** | **Stripping temp, °C** | **Viscosity 40°C** Pa·s **(cP)** | **Mn** | **Mw** | **Mz** | **PDI** |
|---|---|---|---|---|---|---|---|---|
| Once metathesized (1X) | BB9028 | soy oil | 200 | 0.305 (305cP) | NA | NA | NA | NA |
| Once metathesized (1X) | BB9040 | soy oil | 200 | 0.285 (285cP) | 2397 | 5913 | 13273 | 2.5 |
| Twice metathesized (2X) | BB9035 | BB9028 | 150 | 2.93 (2930cP) | 2594 | 31089 | 110887 | 12.0 |
| Twice metathesized (2X) | BB9039 | BB9028 | 200 | 3.244 (3244cP) | 2750 | 31235 | 105716 | 11.4 |
| Twice metathesized (2X) | BB9047A | BB9040 | 150 | 2.66 (2660cP) | NA | NA | NA | NA |
| Twice metathesized (2X) | BB9047B | BB9040 | 200 | 3.05 (3050cP) | 2900 | 30819 | 102295 | 10.6 |
| NOTE: All catayst loading = 38-42 ppm. All reaction temp = 70°C | | | | | | | | |

### Data Set #3 on Metathesized Natural Oil Compositions

| | **Sample no.** | **Feedstock** | **Stripping temp, °C** | **Viscosity 100°C** cm²/s **(cSt)** |
|---|---|---|---|---|
| Once metathesized (1X) | BB8089 | Canola oil | 200 | 0.884 (88.4cSt) |
| Once metathesized (1X) | BB8087 | Canola oil | 200 | 0.837 (83.7cSt) |
| Once metathesized (1X) | BB8092B | Canola oil | 200 | 0.823 (82.3cSt) |
| Twice metathesized (2X) | BB8092D | BB8092B | 200 | 5.499 (549.9cSt) |

### Data Set # 4

### Synthesis of high molecular weight MSBO

In some embodiments, the metathesized natural oil composition is metathesized soybean oil (MSBO). The MSBO was synthesized as follows: Soybean oil (SBO) was charged into a 5 liter reactor with overhead mechanical stirring, nitrogen sparge tube, and nitrogen outlet. The SBO was stirred and sparged with nitrogen for thirty minutes at ambient temperature. The SBO was then heated to 200 °C for two hours, continuously sparging nitrogen. The SBO was cooled to room temperature overnight.

The reaction was stirred and sparged with nitrogen at room temperature for 30 minutes. The sparge tube was pulled above oil level to maintain nitrogen pad during reaction. The SBO was heated to 70 °C. When the reactor reached desired temperature 40 ppm of ruthenium catalyst (C827) was added. The reaction was continued until the oil reached a dynamic viscosity of 0.235 Pa·s (235 cPs) at 25 °C with a spindle speed of 10 RPM. Then, 5 wt% of Oildri B80 clay was added to the reactor, and heated to 80 °C for 1 hour for catalyst removal. The oil / clay mixture was filtered through a sand bed in a pressure filter at 413.685 kPa (60 PSI). Following filtration, the oil was stripped at 200 °C for 2 hours under vacuum.

The stripped MSBO was then loaded into a reactor equipped with a nitrogen sparge tube, overhead mechanical stirring and nitrogen outlet. The MSBO was sparged with stirring at room temperature for 30 minutes. The reactor was heated to 70 °C and 40 ppm of ruthenium catalyst (C827) was added. The reaction was held at temperature until equilibrium, as determined by stable dynamic viscosity samples (taken at 30 minute intervals), was reached.

Several MSBO samples were analyzed using gel permeation chromatography (or GPC as used herein) and dynamic viscosity (or DV as used herein) at 40 °C. The samples (concentration 10 mg/mL in THF) were analyzed using GPC with Agilent 2x Oligopore (300 x 7.5 mm) columns with THF as the eluting solvent at a constant flow rate of 1 mL/min at 35 °C. The GPC system consisted of a Varian Pro-star 210 pump, 410 Auto-sampler, 325 Dual wavelength UV-Vis detector and 356 RI-detector. The molecular weights of the samples were determined using narrow polystyrene (Polymer standard service, Warwick, RI, USA) GPC calibration standards (MW = 17300 -162) in Polymer lab and Cirrus GPC analysis software. Anhydrous THF stabilized with BHTwas purchased from Pharmco-Aaper and used as received. The dynamic viscosity of these samples, shown in Table 1, indicated that 1064-6-4 is of lower conversion. The dynamic viscosity of the stripped material was not lower than the pre-stripped material (1033-96-7). Sample 1064-6-4 was used to generate the target viscosities for high molecular weight MSBO.

**Table 1. Dynamic Viscosity of Retain Samples**

| Sample ID | Description | Dynamic Viscosity at 40 °C 10 RPM Pa·s (cP) |
|---|---|---|
| 1033-96-7 | Pre-stripped 2x MSBO | 0.860 Pa·s (860cP) |
| 1033-96-9 | Stripped 2x MSBO (150 °C) | 1.090 Pa·s (1090cP) |
| 1064-6-4 | Stripped 2x MSBO (150 °C) | 0.695 Pa·s (695cP) |
| BB9004 | Pre-stripped 2x MSBO | 0.740 Pa·s (740cP) |
| BB9004S | Stripped 2x MSBO (200 °C) | 1.360 Pa·s (1360cP) |

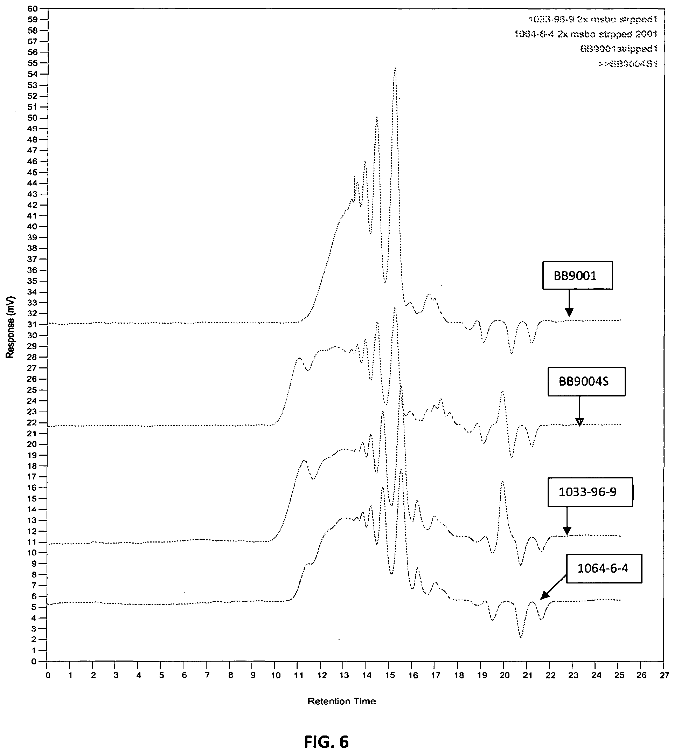

After observing the discontinuity in dynamic viscosity, GPC was utilized to compare the molecular weight distributions. The results are shown in Figure 6, which depicts a gel permeation chromatogram of high molecular weight MSBO samples. For comparison, sample BB9001 was included as a reference to MSBO, the starting material. The GPC data shows that Sample 1064-6-4 has less higher molecular weight fractions versus samples 1033-96-9 and BB9007, indicating that 1064-6-4 is lower conversion then other batches of high molecular weight MSBO. As understood, higher conversions will produce higher molecular weight polymers.

### Kinetic study of high molecular weight MSBO

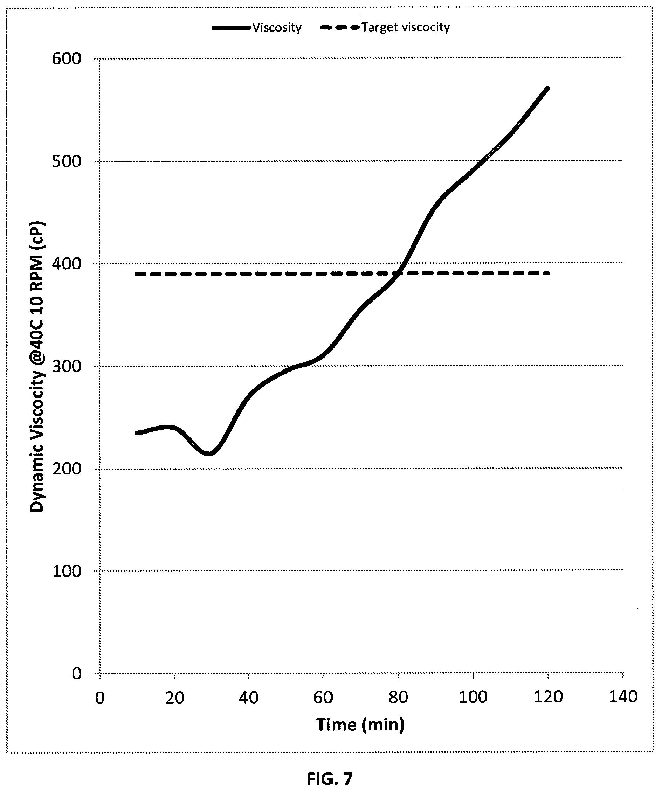

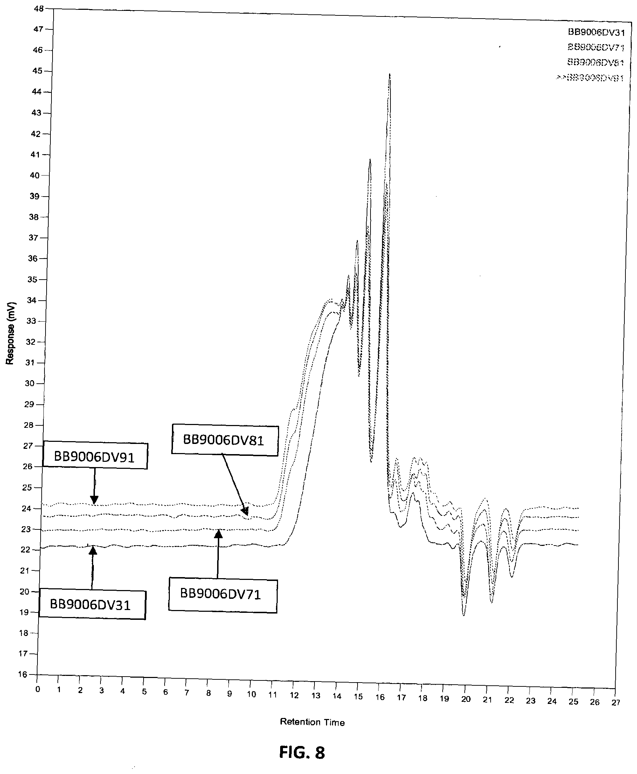

360 g of high molecular weight MSBO was synthesized in a 500 mL flask. The reaction was carried out using the same method as describe in the previous section, with the exception of catalyst loading and stirring with a magnetic stir bar. In an attempt to slow the reaction down catalyst was loaded at 30 ppm. Samples were taken every ten minutes after catalyst was added. Samples were analyzed using dynamic viscosity and GPC. The kinetic study for high molecular weight MSBO was not taken to equilibrium, as the purpose of this study was to determine the time point at which the target viscosity of 0.390 Pa·s (390 cP) at 40 °C was achieved. Dynamic viscosity analyses of kinetic samples of high molecular weight MSBO were graphed in reference to time, as shown in Figure 7, and the relevant data is shown in Table 2. This study showed that the target viscosity was reached in 80 minutes. This did not directly translate to larger scale. Upon scale up to the 5 L reactor, the batch reached the targeted viscosity in 60 minutes. This could be due to different stirring capabilities. The kinetic study was performed using magnetic stirring while larger scale reactions utilize overhead mechanic stirring. GPC was also used to analyze kinetic samples for molecular weight distributions as shown in Figure 8 (GPC overlay of kinetic studies of high molecular weight MSBO with 30 ppm catalyst; BB9006DV31 @ 30 minutes, BB9006DV71 @ 70 minutes, BB9006DV81 @ 80 minutes, and BB9006DV91 @ 90 minutes). This shows that the higher molecular weight fraction is achieved over time. Through further study, GPC could be used as an analytical method to determine reaction completion in addition to dynamic viscosity.

**Table 2**

| Kinetic study time (min) | 30 ppm catalyst loading level DV @40°C 10RPM |
|---|---|
| 0 | 230 |
| 10 | 235 |
| 20 | 240 |
| 30 | 215 |
| 40 | 270 |
| 50 | 295 |
| 60 | 310 |
| 70 | 355 |
| 80 | 390 |
| 90 | 455 |
| 100 | 490 |
| 110 | 525 |
| 120 | 570 |

### Blending Study of high molecular weight MSBO and MSBO

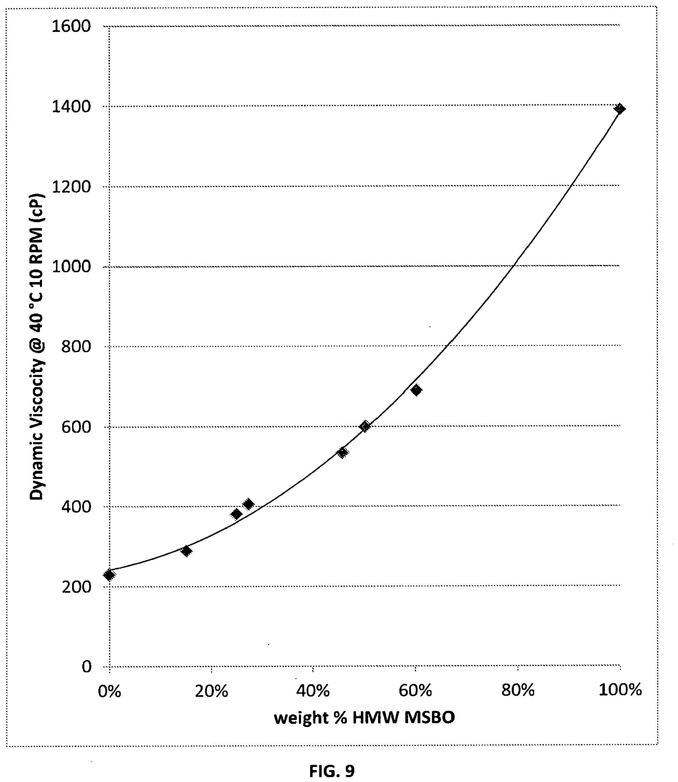

High molecular weight MSBO was blended with MSBO to determine the feasibility of back blending to hit target viscosities. The initial blend was estimated using the weighted average viscosity of the blend. After the initial blend was measured, further blends were adjusted to the target viscosity. Figure 9 below shows the blending properties of high molecular weight MSBO (BB9004) back blended with MSBO (BB9001), and Table 3 shows the relevant data. When blended, the viscosity did not follow a linear trend. In order to achieve the target viscosity for certain samples (Sample 1064-6-4), MSBO would have to be blended with 45-50 wt% high molecular weight MSBO. Note: this blending study was done using equilibrated high molecular weight MSBO, not lower conversion high molecular weight MSBO as will be discussed in the following section.

**Table 3**

| Blending study wt% 2x in MSBO | bb9004 DV@40°C 10 RPM |
|---|---|
| 0.00% | 230 |
| 15.18% | 290 |
| 24.91% | 380 |
| 27.23% | 405 |
| 45.84% | 535 |
| 50.28% | 600 |
| 60.30% | 690 |
| 100.00% | 1390 |

### Synthesis of lower conversion high molecular weight MSBO

Using the results of the kinetic study and the blending study, a low conversion, high molecular weight MSBO, targeting 0.390 Pa·s (390 cP) at 40 °C with a speed of 10 RPM, was synthesized. Using 30 ppm ruthenium catalyst (C827), and the time estimate determined by the kinetic study, high molecular weight MSBO was synthesized. During the reaction, samples were taken and analyzed using dynamic viscosity to determine reaction progression, the results of which are listed in Table 4. When the target viscosity was reached, the reaction was stopped by the addition of B80 clay and heating to 80 °C. For previous studies of MSBO, the initial rise in viscosity was slow, and as conversion increases, viscosity increased at a faster rate. The extra time, past the target viscosity, was due to the time needed to measure the dynamic viscosity.

**Table 4. Dynamic Viscosity data for kinetic samples**

| Time (Min) | Dynamic Viscosity at 40 °C 10 RPM |
|---|---|
| 0 | 200 |
| 40 | 285 |
| 60 | 390 |
| 75 | 600 |

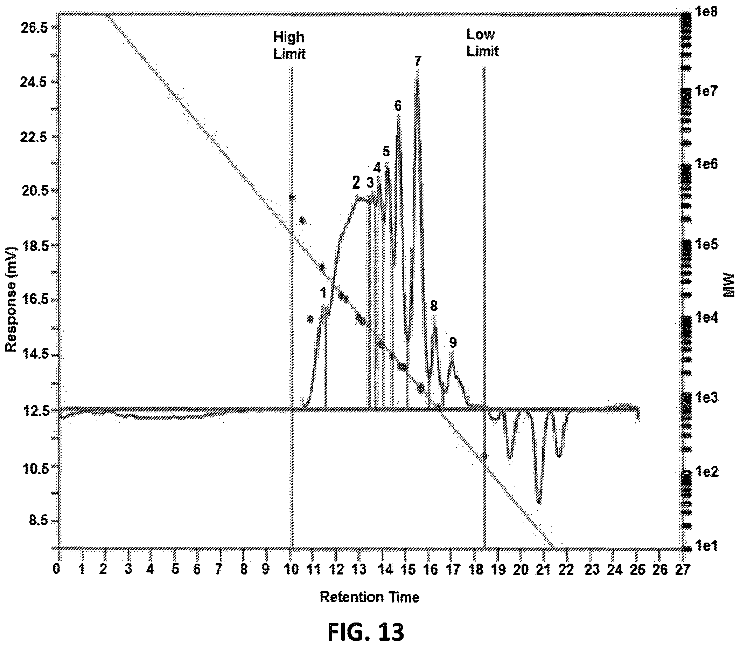

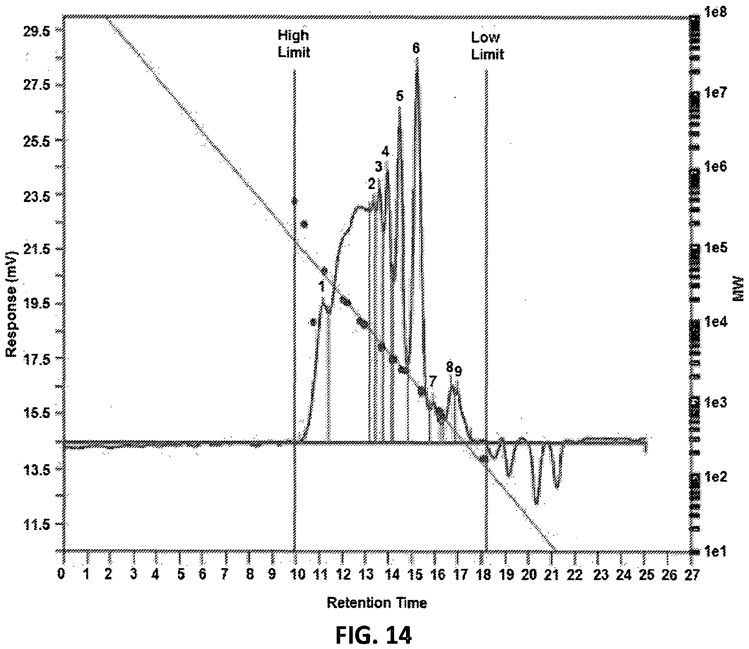

The viscosity of the 150 °C stripped sample was 0.855 Pa·s (855 cP) at 40 °C, where the target viscosity for this sample was 0.567 Pa·s (567 cP). In order to achieve the target viscosity, the sample was back blended with 24.9 wt% MSBO. This yielded a sample (BB9014) with a viscosity of 0.590 Pa·s (590 cP) at 40 °C with a speed of 10 RPM. GPC was run on this blend sample for comparison to sample LF 2XMSBO @150°C, and the GPC reports showed similar profiles, as shown in Figure 13 (for BB9014) and Figure 14 (for LF 2XMSBO), with molecular weight averages as shown in Table 5. The LF2XMSBO sample contained a higher concentration of the high molecular weight fraction than the blended sample, and the LF2XMSBO had a viscosity of 0.720 Pa·s (720 cP).

**Table 5a. BB9014**

| Peak No | Mp | Mn | Mw | Mz | Mz+1 | Mv | PD |
|---|---|---|---|---|---|---|---|
| 1 | 46368 | 49688 | 51797 | 54892 | 58172 | 51312 | 1.04244 |
| 2 | 8652 | 14492 | 17049 | 20183 | 23180 | 16595 | 1.17644 |
| 3 | 7170 | 7408 | 7128 | 7301 | 7323 | 7061 | 0.962203 |
| 4 | 5692 | 5766 | 5649 | 5764 | 5801 | 5611 | 0.979709 |
| 5 | 4221 | 4183 | 4140 | 4219 | 4256 | 4117 | 0.98972 |
| 6 | 2788 | 2718 | 2739 | 2797 | 2840 | 2726 | 1.00773 |
| 7 | 1434 | 1410 | 1441 | 1481 | 1517 | 1434 | 1.02199 |
| a | 790 | 781 | 776 | 793 | 802 | 772 | 0.993598 |
| 9 | 407 | 433 | 428 | 437 | 441 | 426 | 0.988453 |
| 10 | 339 | 315 | 308 | 314 | 316 | 306 | 0.977778 |

**Table 5b. LF2XMSBO**

| Peak No | Mp | Mn | Mw | Mz | Mz+1 | Mv | PD |
|---|---|---|---|---|---|---|---|
| 1 | 43346 | 46665 | 48057 | 50257 | 52543 | 47698 | 1.02983 |
| 2 | 7013 | 7293 | 7054 | 7214 | 7242 | 6994 | 0.967229 |
| 3 | 5700 | 5722 | 5585 | 5698 | 5728 | 5545 | 0.976057 |
| 4 | 4258 | 4219 | 4173 | 4251 | 4287 | 4151 | 0.989097 |
| 5 | 2832 | 2776 | 2793 | 2847 | 2887 | 278 1 | 1.00612 |
| 6 | 1473 | 1458 | 1489 | 1526 | 1561 | 1482 | 1.02126 |
| 7 | 826 | 800 | 797 | 813 | 822 | 793 | 0.99625 |
| 8 | 430 | 450 | 448 | 457 | 463 | 446 | 0.995556 |
| 9 | 342 | 310 | 312 | 319 | 323 | 310 | 1.00645 |

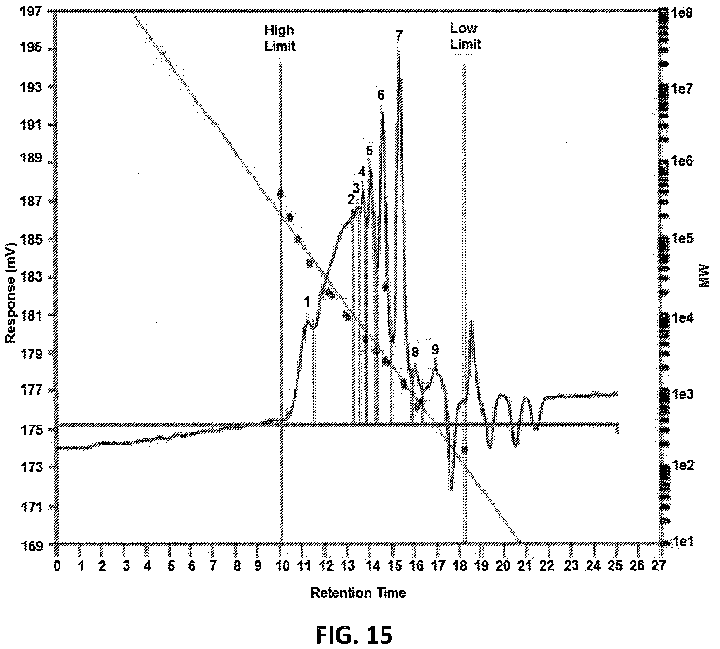

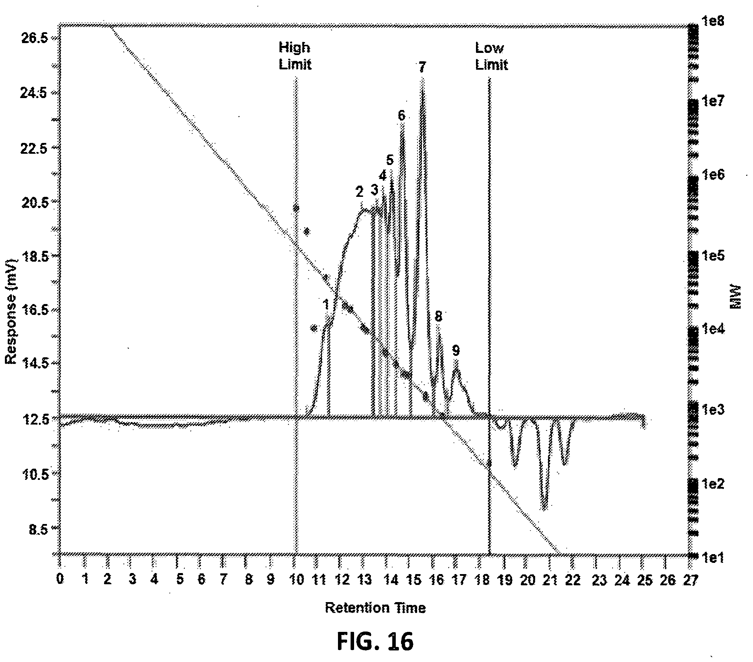

Another sample stripped at 200 °C also had to be back blended due to overshooting viscosity. After stripping at 200 °C the sample had a viscosity of 1.01 Pa·s (1010 cP) at 40 °C with a speed of 10 RPM, and 14.4 wt% MSBO was back blended into the sample to yield a sample (BB9015) with a viscosity of 0.635 Pa·s (635 cP) at 40 °C at 10 RPM. This was close to the target viscosity of 0.650 Pa·s (650 cP). GPC was run on the sample for comparison to sample 1064-6-4. These GPC reports had similar profiles, and similar viscosities, as shown in Figure 15 (for BB 9015) and Figure 16 (for 1064-6-4). Molecular weight averages for these samples are shown in Table 6.

**Table 6a. BB9015**

| **MW Averages** | | | | | | | |
|---|---|---|---|---|---|---|---|
| Peak No | Mp | Mn | Mw | Mz | Mz+1 | Mv | PD |
| 1 | 68987 | 75920 | 80353 | 86672 | 93627 | 79407 | 1.05839 |
| 2 | 10718 | 19255 | 23376 | 28528 | 33419 | 22639 | 1.21402 |
| 3 | 8781 | 9185 | 8902 | 9112 | 9158 | 8827 | 0.969139 |
| 4 | 6858 | 6953 | 6802 | 6950 | 6998 | 6753 | 0.978283 |
| 5 | 4949 | 4908 | 4869 | 4972 | 5027 | 4840 | 0.992054 |
| 6 | 3123 | 3042 | 3076 | 3151 | 3211 | 3061 | 1.01118 |
| 7 | 1492 | 1462 | 1504 | 1554 | 1601 | 1496 | 1.02873 |
| 8 | 791 | 737 | 732 | 750 | 759 | 728 | 0.093216 |
| 9 | 363 | 354 | 372 | 396 | 417 | 369 | 1.05085 |

**Table 6b. 1064-6-4**

| Peak No | Mp | Mn | Mw | Mz | Mz+1 | Mv | PD |
|---|---|---|---|---|---|---|---|
| 1 | 41456 | 46793 | 47555 | 49235 | 50863 | 47250 | 1.01628 |
| 2 | 10772 | 14285 | 116937 | 20175 | 23284 | 16470 | 1.18565 |
| 3 | 6912 | 7099 | 6860 | 7018 | 7044 | 6801 | 0.966333 |
| 4 | 5563 | 5560 | 5431 | 5541 | 5571 | 5393 | 0.976799 |
| 5 | 41 22 | 4077 | 4035 | 4110 | 4146 | 4013 | 0.989698 |
| 6 | 2719 | 2668 | 2685 | 2738 | 2778 | 2674 | 1.00637 |
| 7 | 1388 | 1377 | 1406 | 1443 | 1477 | 1400 | 1.02106 |
| a | 766 | 743 | 746 | 758 | 767 | 743 | 1.00404 |
| 9 | 401 | 359 | 372 | 387 | 400 | 370 | 1.03621 |

Table 7 below states the reaction conditions for several of the lower conversion high molecular weight MSBO samples.

**Table 7**

| **MSBO** | | **LF DV@40 Pa·s (cPs)** | **BB DV@40 (BB9001/04) Pa·s (cPs)** | **wfe74 to bb8174** | **DV@40 Sample ID (% stripped)** | | **DV@40 Comment** | |
|---|---|---|---|---|---|---|---|---|
| 1x unstripped (135cP) | | 0.135 (135cP) | 0.115 (115cP) | | | | | |
| 1x @200 (305cP) | | 0.305 (305cP) | 0.230 (230cP) | 285 | 200 BB9010(12.5) | | | |
| 2x unstripped (392.5cP) | | 0.3925 (392.5cP) | 0740 (740cP) | 1390 | 600 BB9010 | | | |
| 2x @150 | | 0.5675 (567.5cP) | | | 855 BB9012(4.0) | | BB9014 (75.1% BB9012 590 w/ BB9010) | |
| 2x @200 | | 0.65 (650cP) | 1.360 (1360cP) | | 1010 BB9013(6.1) | | BB9015 (85.6% BB9013 635 w/BB9010) | |
| | | | | | | | | |

| | **1X BB9011** | **1X WFE74** | **2X BB8174** | **2X BB9004** | **1X BB9010** | **2x unstripped BB9011** | **2X BB9013** | **2X BB9012** |
|---|---|---|---|---|---|---|---|---|
| Reactant | SBO | SBO | WFE74 | BB9001 | SBO | BB9010 | BB9011 | BB9011 |
| **PV** | 0.23 | <1 | | | 0.23 | | | |
| **Batch size, g** | 3117 | -14000 | 1325.7 | 1417.8 | 4230.6 | 1516.8 | | |
| **C827 load,** ppm | 39 | 40 | 66 | 60 | 43 | 30 | | |
| **Rxn temp,** °C | 67 | 67 | 67 | 67 | 70 | 67.5 | | |
| **Rxn time, min** | 255 | 120 | 240 | 108 | 300 | 75 | | |
| **% conversion** | 75.6 | 72-83 | 72.88 | | 70.6 | | | |
| **Unstrip visc, DV@ 25°C** | 230 | 205 | | | 200 | | | |
| **Unstrip visc, DV@ 40°C** | 115 | | 1390 | 740 | | 600 | 600 | 600 |
| **Strip temp, °C** | 200 | 200 | 200 | 200 | 200 | | 200 | 150 |
| **Strip time, min** | 120 | 2-passes | 205 | 150 | 240 | | 240 | 120 |
| **Wt%** stripped | 12.38 | 11-12 | 5 | 8.07 | 12.5 | | 6.08 | 4 |
| **Strip visc, DV@40°C** | 230 | 285 | | 1360 | 200 | | 1010 | 855 |

### Data Set # 5

### Synthesis of high molecular weight MSBO and kinetic studies

Two batches of MSBO were synthesized as described in Data Set #4. The MSBO was then loaded into a 5 L reactor equipped with a nitrogen sparge tube, overhead mechanical stirring and nitrogen outlet. The MSBO was sparged while stirring at room temperature for 30 minutes. The reactor was heated to 70 °C and the sparging tube was raised above oil level to keep positive nitrogen pressure. Then, 40 ppm of ruthenium catalyst (C827) was added to the reactor. The reaction was held at temperature until equilibrium, as determined by stable dynamic viscosity samples taken at 30-minute intervals, was reached.

Kinetic samples were removed from the reactor and placed into a freezer to stop the reaction progress. When the sample's temperature was below 40 °C, dynamic viscosity was measured. After the dynamic viscosity was determined, the sample was placed back into freezer. Upon completion of the reaction, all kinetic samples were removed from the freezer to prepare GPC samples.

Synthesis of high molecular weight MSBO using standard metathesis conditions yielded a product with a pre-stripped viscosity between 1.51-1.61 Pa·s (1510-1610 cP) at 40 °C and 10 RPM. The products viscosity was dependent on the starting MSBO'S viscosity. A lower viscosity starting material will produce a lower viscosity product. A percent difference of 6.8% between MSBO batches led to a 9.5% difference in the pre-stripped high molecular weight MSBO. The difference in the MSBO viscosity was due to 0.4 wt% difference in stripping levels.

Stripping of high molecular weight MSBO was done at 150 °C and 200 °C to show difference in products due to stripping level. Stripping at 150 °C for 2 hours was sufficient to remove any benzene made during the metathesis reaction. Comparison of stripped products was more difficult, as the high molecular weight MSBO was not stripped of the same wt%, and small changes in stripping can have a significant impact on viscosity. The reaction conditions of and viscosities of various MSBO and high molecular weight MSBO products are shown in Table 8 below.

**Table 8**

| | Standard Batch 1 | | | | Standard Batch 2 | | | |
|---|---|---|---|---|---|---|---|---|
| | **BB9028/33** | **BB9033DV6** | **BB9035** | **BB9039** | **BB9040** | **BB9042** | **BB9047A** | **BB9047B** |
| reactant/ starting material | SBO | BB9033 | BB9033DV6 | BB9033DV6 | SBO | BB9040 | BB9042 | BB9042 |
| Batch size, g | 2926.3 | 2266.7 | - | - | 3453 | 2562.8 | - | - |
| C827 load, ppm | 42 | 42.2 | - | - | 38 | 42 | - | - |
| rxn temp, C | 70 | 70 | - | - | 70 | 70 | - | - |
| rxn duration, min | 210 | 190 | - | - | 240 | 210 | - | - |
| % conversion | 71.96 | - | - | - | 71.33 | - | - | - |
| unstrip visc, DV, 25C | 235 | - | - | - | 235 | - | - | - |
| unstrip visc, DV, 40C | | 1660 | 1660 | 1660 | - | 1510 | - | - |
| strip temp, C | 200 | - | 150 | 200 | 200 | - | 150 | 200 |
| strip duration, min | 120 | - | 120 | 120 | 120 | - | 120 | 180 |
| wt % stripped | 13.3 | - | 6.5 | 7.5 | 12.9 | - | 5.8 | 7.3 |
| strip visc, DV, 40C | 305 | - | 2930 | 3244 | 285 | - | 2660 | 3050 |

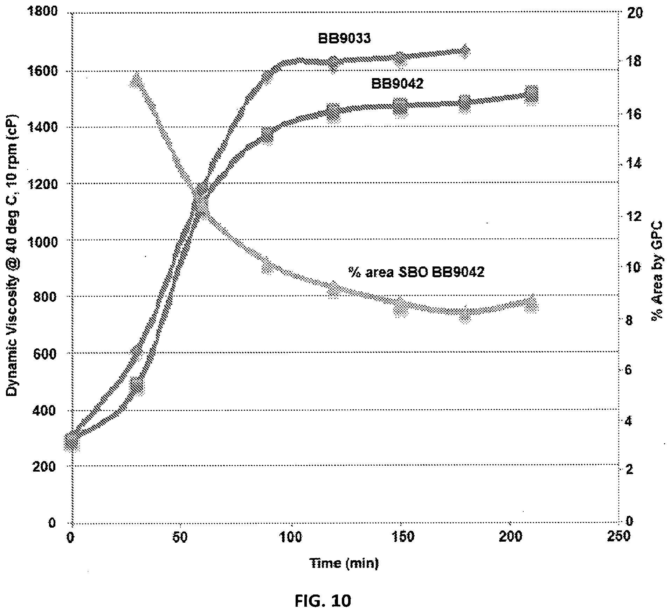

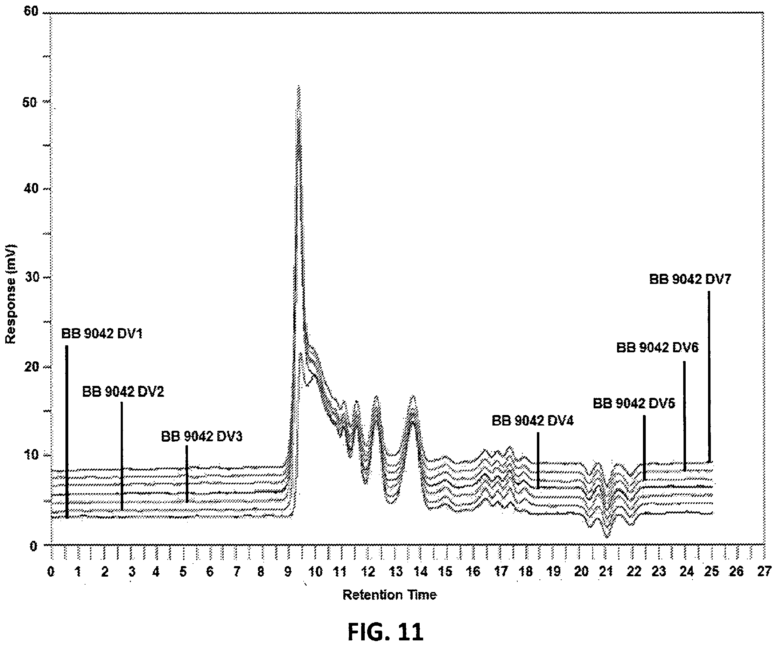

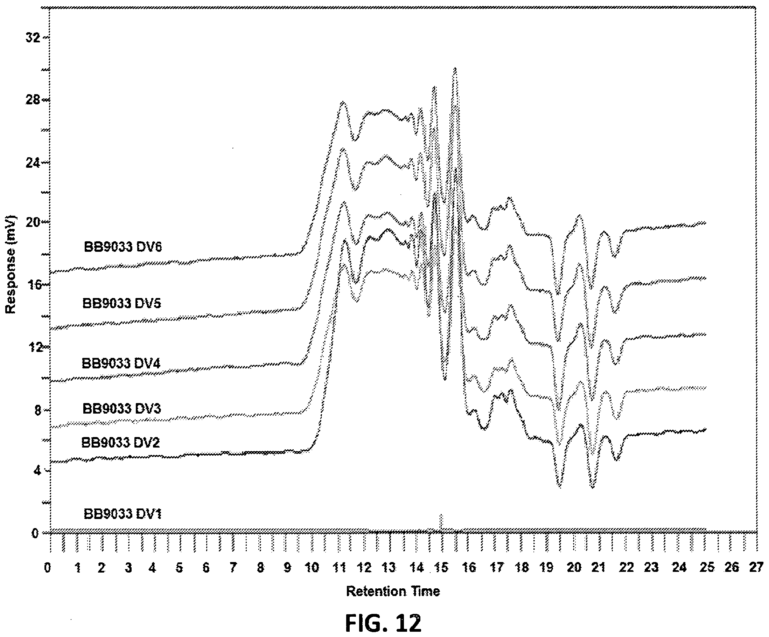

Kinetic samples were evaluated using both GPC and with dynamic viscosity at 40 °C. The dynamic viscosity was plotted versus time as shown in Figure 10, with the relevant data shown in Table 9. This graph shows a similar rate of reaction, as both batches of high molecular weight MSBO have a similar slope. Between standard batch 1 (Figure 12) and standard batch 2 (Figure 11), the columns in the GPC were replaced and better separation was observed. Standard batch 1 was not re-run on the new columns as they had been at room temperature and metathesis may have slowly continued, thus skewing data. Improved resolution allowed the tracking of the SBO as the reaction progressed and was plotted with dynamic viscosity measurements, shown in Figure 10. The peak area of the soybean oil was inversely related to the dynamic viscosity and appeared to equilibrate at the same time as the viscosity.

**Table 9**

| Time (min) | BB9033 Dynamic Viscosity @40° C 10RPM | %change | BB9042 Dynamic Viscosity @40° C 10RPM | %change | % area SBO | % area HMW peak |
|---|---|---|---|---|---|---|
| 0 | 305 | | 285 | --- | --- | --- |
| 30 | 605 | 98.36065574 | 485 | 70.1754386 | 17.4619 | 40.7992 |
| 60 | 1180 | 95.04132231 | 1115 | 129.8969072 | 12.4292 | 61.1934 |
| 90 | 1580 | 33.89830508 | 1375 | 23.31838565 | 10.1885 | 69.73618 |
| 120 | 1620 | 2.53164557 | 1450 | 5.454545455 | 9.20819 | 71.4283 |
| 150 | 1635 | 0.925925926 | 1470 | 1.379310345 | 8.5628 | 73.29161 |
| 180 | 1660 | 1.529051988 | 1480 | 0.680272109 | 8.23834 | 74.85521 |
| 210 | | | 1510 | 2.027027027 | 8.65227 | 73.71407 |

## Claims

1. A metathesized natural oil composition comprising a metathesized natural oil, wherein the metathesized natural oil composition has a number average molecular weight in the range from 2,000 g/mol to 4,000 g/mol, a weight average molecular weight in the range from 7,000 g/mol to 35,000 g/mol, a z-average molecular weight in the range from 10,000 g/mol to 125,000 g/mol, and a polydispersity index of 2 to 12, a dynamic viscosity at 40°C of between 0.2 Pa·s (200 centipoise) and 3.5 Pa·s (3500 centipoise), and a dynamic viscosity at 100°C of between 0.075 Pa·s (75 centipoise) and 0.6 Pa·s (600 centipoise).

2. The metathesized natural oil composition of claim 1, wherein the metathesized natural oil composition has a number average molecular weight in the range from 2,300 g/mol to 3,400 g/mol, a weight average molecular weight in the range from 9,600 g/mol to 31,500 g/mol, and a z-average molecular weight in the range from 28,000 g/mol to 111,000 g/mol, and a polydispersity index of 4 to 12.

3. The metathesized natural oil composition of claim 1, wherein the metathesized natural oil composition has a dynamic viscosity at 40°C of between 0.55 Pa·s (550 centipoise) and 3.25 Pa·s (3250 centipoise).

4. The metathesized natural oil composition of any of claims 1 to 3, wherein the metathesized natural oil composition is made by reacting a natural oil feedstock in the presence of a metathesis catalyst to form a first metathesized natural oil product, and further wherein the first metathesized natural oil product is self-metathesized to form another metathesized natural oil product.

5. The metathesized natural oil composition of any of claims 1 to 3, wherein the metathesized natural oil composition is made by reacting a natural oil in the presence of a metathesis catalyst to form a first metathesized natural oil product, and further wherein the first metathesized natural oil product is cross-metathesized with a natural oil to form another metathesized natural oil product.

6. The metathesized natural oil composition of any of claims 1 to 3, wherein the metathesized natural oil composition may be subject to steam stripping at a temperature of between 100 °C and 250 °C.

7. The metathesized natural oil composition of any of claims 1 to 3, wherein the metathesized natural oil composition may be subject to reduced pressure conditions to remove C12 or lighter olefins.

8. The metathesized natural oil composition of any of claims 1 to 3, wherein when the metathesized natural oil composition comprises a mixture of olefins and esters, and further wherein the mixture of olefins and esters can be separated into olefins and esters, and thereafter transesterifying the esters in the presence of an alcohol to form a transesterified product.

9. The metathesized natural oil composition of any of claims 1 to 3, wherein the molecular weight of the metathesized natural oil composition can be increased by transesterifying the metathesized natural oil composition with diesters.

10. The metathesized natural oil composition of any of claims 1 to 3, wherein the molecular weight of the metathesized natural oil composition can be increased by transesterifying the metathesized natural oil composition with diacids.

11. The metathesized natural oil composition of any of claims 1 to 3, wherein the metathesized natural oil in the metathesized natural oil composition is selected from the group consisting of metathesized canola oil, metathesized rapeseed oil, metathesized coconut oil, metathesized corn oil, metathesized cottonseed oil, metathesized olive oil, metathesized palm oil, metathesized peanut oil, metathesized safflower oil, metathesized sesame oil, metathesized soybean oil, metathesized sunflower oil, metathesized linseed oil, metathesized palm kernel oil, metathesized tung oil, metathesized jatropha oil, metathesized mustard oil, metathesized castor oil, metathesized camelina oil, metathesized pennycress oil, metathesized derivatives of these oils, and mixtures thereof.

12. The metathesized natural oil composition of any of claims 1 to 3, wherein the metathesized natural oil composition may be utilized independently and/or incorporated into various formulations and used as functional ingredients, selected from the group consisting of dimethicone replacements, laundry detergents, fabric softeners, emollients, hair fixative polymers, rheology modifiers, specialty conditioning polymers, surfactants, UV absorbers, solvents, humectants, occlusives, cosmetics, lip balms, lipsticks, hair dressings, sun care products, moisturizer, fragrance sticks, perfume carriers, skin feel agents, shampoos/conditioners, bar soaps, hand soaps/washes, bubble baths, body washes, facial cleansers, shower gels, wipes, baby cleansing products, creams/lotions, antiperspirants/deodorants, lubricants, functional fluids, fuels and fuel additives, additives for such lubricants, functional fluids and fuels, plasticizers, asphalt additives, friction reducing agents, antistatic agents in the textile and plastics industries, flotation agents, gelling agents, epoxy curing agents, corrosion inhibitors, pigment wetting agents, in cleaning compositions, plastics, coatings, adhesives, skin feel agents, film formers, rheological modifiers, release agents, conditioners dispersants, hydrotropes, industrial and institutional cleaning products, oil field applications, gypsum foamers, sealants, agricultural formulations, enhanced oil recovery compositions, solvent products, gypsum products, gels, semi-solids, detergents, heavy duty liquid detergents, light duty liquid detergents, liquid detergent softeners, antistat formulations, dryer softeners, hard surface cleaners for household, autodishes, rinse aids, laundry additives, carpet cleaners, softergents, single rinse fabric softeners, oven cleaners, car washes, transportation cleaners, drain cleaners, defoamers, anti- foamers, foam boosters, anti-dust/dust repellants, industrial cleaners, institutional cleaners, janitorial cleaners, glass cleaners, graffiti removers, concrete cleaners, metal/machine parts cleaners, pesticides, agricultural formulations and food service cleaners, plasticizers, asphalt additives and emulsifiers, friction reducing agents, film formers, rheological modifiers, biocides, biocide potentiators, release agents, household cleaning products, including liquid and powdered laundry detergents, liquid and sheet fabric softeners, hard and soft surface cleaners, sanitizers and disinfectants, and industrial cleaning products, emulsion polymerization, including processes for the manufacture of latex and for use as surfactants as wetting agents, formulation inerts in pesticide applications or as adjuvants used in conjunction with the delivery of pesticides including agricultural crop protection turf and ornamental, home and garden, and professional applications, institutional cleaning products, oil field applications, including oil and gas transport, production, stimulation and drilling chemicals and reservoir conformance and enhancement, organoclays for drilling muds, specialty foamers for foam control or dispersancy in the manufacturing process of gypsum, cement wall board, concrete additives and firefighting foams, paints and coalescing agents, paint thickeners, or other applications requiring cold tolerance performance or winterization.

13. The metathesized natural oil composition of claim 1 comprising (i) wherein the metathesized natural oil composition has a number average molecular weight in the range from 2,600 g/mol to 3,100 g/mol, a weight average molecular weight in the range from 12,000 g/mol to 13,500 g/mol, and a z-average molecular weight in the range from 38,000 g/mol to 46,000 g/mol, and a polydispersity index of 4 to 5, and further wherein the metathesized natural oil composition is metathesized at least twice.

14. The metathesized natural oil composition of claim 13, wherein the metathesized natural oil composition is subject to steam stripping at a temperature of between 150° C and 200° C, and has a dynamic viscosity at 40°C of between 0.575 Pa·s (575 centipoise) and 0.67 Pa·s (670 centipoise).

## Patentansprüche

1. Eine Zusammensetzung metathesierten natürlichen Öls, beinhaltend ein metathesiertes natürliches Öl, wobei die Zusammensetzung metathesierten natürlichen Öls ein Molekulargewicht im Zahlenmittel in dem Bereich von 2 000 g/mol bis 4 000 g/mol, ein Molekulargewicht im Gewichtsmittel in dem Bereich von 7 000 g/mol bis 35 000 g/mol, ein z-durchschnittliches Molekulargewicht in dem Bereich von 10 000 g/mol bis 125 000 g/mol und einen Polydispersitätsindex von 2 bis 12, eine dynamische Viskosität bei 40 °C von zwischen 0,2 Pa·s (200 Centipoise) und 3,5 Pa·s (3 500 Centipoise), und eine dynamische Viskosität bei 100 °C von zwischen 0,075 Pa·s (75 Centipoise) und 0,6 Pa·s (600 Centipoise) aufweist.

2. Zusammensetzung metathesierten natürlichen Öls gemäß Anspruch 1, wobei die Zusammensetzung metathesierten natürlichen Öls ein Molekulargewicht im Zahlenmittel in dem Bereich von 2 300 g/mol bis 3 400 g/mol, ein Molekulargewicht im Gewichtsmittel in dem Bereich von 9 600 g/mol bis 31 500 g/mol und ein z-durchschnittliches Molekulargewicht in dem Bereich von 28 000 g/mol bis 111 000 g/mol und einen Polydispersitätsindex von 4 bis 12 aufweist.

3. Zusammensetzung metathesierten natürlichen Öls gemäß Anspruch 1, wobei die Zusammensetzung metathesierten natürlichen Öls eine dynamische Viskosität bei 40 °C von zwischen 0,55 Pa·s (550 Centipoise) und 3,25 Pa·s (3 250 Centipoise) aufweist.

4. Zusammensetzung metathesierten natürlichen Öls gemäß einem der Ansprüche 1 bis 3, wobei die Zusammensetzung metathesierten natürlichen Öls hergestellt wird durch Zur-Reaktion-Bringen eines Ausgangsmaterials aus natürlichem Öl in Gegenwart eines Metathesekatalysators, um ein erstes Produkt metathesierten natürlichen Öls zu bilden, und wobei ferner das erste Produkt metathesierten natürlichen Öls selbstmetathesiert wird, um ein weiteres Produkt metathesierten natürlichen Öls zu bilden.

5. Zusammensetzung metathesierten natürlichen Öls gemäß einem der Ansprüche 1 bis 3, wobei die Zusammensetzung metathesierten natürlichen Öls hergestellt wird durch Zur-Reaktion-Bringen eines natürlichen Öls in Gegenwart eines Metathesekatalysators, um ein erstes Produkt metathesierten natürlichen Öls zu bilden, und wobei ferner das erste Produkt metathesierten natürlichen Öls mit einem natürlichen Öl kreuzmetathesiert wird, um ein weiteres Produkt metathesierten natürlichen Öls zu bilden.

6. Zusammensetzung metathesierten natürlichen Öls gemäß einem der Ansprüche 1 bis 3, wobei die Zusammensetzung metathesierten natürlichen Öls einer Wasserdampfdestillation bei einer Temperatur von zwischen 100 °C und 250 °C unterzogen werden kann.

7. Zusammensetzung metathesierten natürlichen Öls gemäß einem der Ansprüche 1 bis 3, wobei die Zusammensetzung metathesierten natürlichen Öls Bedingungen reduzierten Drucks unterzogen werden kann, um C12-Olefine oder leichtere Olefine zu entfernen.

8. Zusammensetzung metathesierten natürlichen Öls gemäß einem der Ansprüche 1 bis 3, wobei, wenn die Zusammensetzung metathesierten natürlichen Öls eine Mischung von Olefinen und Estern beinhaltet, und wobei ferner die Mischung von Olefinen und Estern in Olefine und Ester getrennt werden kann, und danach Umestern der Ester in Gegenwart eines Alkohols, um ein umgeestertes Produkt zu bilden.

9. Zusammensetzung metathesierten natürlichen Öls gemäß einem der Ansprüche 1 bis 3, wobei das Molekulargewicht der Zusammensetzung metathesierten natürlichen Öls durch Umestern der Zusammensetzung metathesierten natürlichen Öls mit Diestern erhöht werden kann.

10. Zusammensetzung metathesierten natürlichen Öls gemäß einem der Ansprüche 1 bis 3, wobei das Molekulargewicht der Zusammensetzung metathesierten natürlichen Öls durch Umestern der Zusammensetzung metathesierten natürlichen Öls mit zweiwertigen Säuren erhöht werden kann.

11. Zusammensetzung metathesierten natürlichen Öls gemäß einem der Ansprüche 1 bis 3, wobei das metathesierte natürliche Öl in der Zusammensetzung metathesierten natürlichen Öls ausgewählt ist aus der Gruppe, bestehend aus metathesiertem Canola-Rapsöl, metathesiertem Rapsöl, metathesiertem Kokosnussöl, metathesiertem Maisöl, metathesiertem Baumwollsamenöl, metathesiertem Olivenöl, metathesiertem Palmöl, metathesiertem Erdnussöl, metathesiertem Distelöl, metathesiertem Sesamöl, metathesiertem Sojabohnenöl, metathesiertem Sonnenblumenöl, metathesiertem Leinsamenöl, metathesiertem Palmkernöl, metathesiertem Tungöl, metathesiertem Jatrophaöl, metathesiertem Senföl, metathesiertem Rizinusöl, metathesiertem Leindotteröl, metathesiertem Hellerkrautöl, metathesierten Ableitungen dieser Öle und Mischungen davon.

12. Zusammensetzung metathesierten natürlichen Öls gemäß einem der Ansprüche 1 bis 3, wobei die Zusammensetzung metathesierten natürlichen Öls unabhängig genutzt und/oder in verschiedenen Formulierungen eingearbeitet und als funktionelle Bestandteile verwendet werden kann, ausgewählt aus der Gruppe, bestehend aus Dimeticonersätzen, Waschmitteln, Weichspülern, Emollientien, Haarfixierungspolymeren, Rheologiemodifikatoren, Konditionierungspolymeren für Spezialanwendungen, Tensiden, UV-Absorbern, Lösungsmitteln, Benetzungsmitteln, Abdeckmitteln, Kosmetika, Lippenbalsamen, Lippenstiften, Haarpflegemitteln, Sonnenpflegeprodukten, Feuchtigkeitsspendern, Duftstäben, Parfümträgern, Hautgefühlmitteln, Shampoos/Conditionern, Seifen in Stücken, Handseifen/-wäschen, Schaumbädern, Duschlotionen, Gesichtsreinigern, Duschgelen, Wischtüchern, Babyreinigungsprodukten, Cremes/Lotionen, Antitranspirants/Deodorants, Schmiermitteln, funktionellen Fluiden, Brennstoffen und Brennstoffzusätzen, Zusätzen für derartige Schmiermittel, funktionelle Fluide und Brennstoffe, Weichmachern, Asphaltzusätzen, Reibung reduzierenden Mitteln, antistatischen Mitteln in der Textil- und Kunststoffindustrie, Flotationsmitteln, Geliermitteln, Epoxid härtenden Mitteln, Korrosionsinhibitoren, Pigmentbenetzungsmitteln, in Reinigungszusammensetzungen, Kunststoffen, Beschichtungen, Klebstoffen, Hautgefühlmitteln, Filmbildnern, Rheologiemodifikatoren, Trennmitteln, Konditionierern, Dispergiermitteln, hydrotropen Stoffen, industriellen und institutionellen Reinigungsprodukten, Ölfeldanwendungen, Gipsschaumbildnern, Dichtungsmitteln, landwirtschaftlichen Formulierungen, Zusammensetzungen zur gesteigerten Ölrückgewinnung, Lösungsmittelprodukten, Gipsprodukten, Gelen, halbfesten Stoffen, Detergenzien, flüssigen Starkreinigern, flüssigen Feinwaschmitteln, flüssigen Detergens-Weichspülern, antistatischen Formulierungen, Trocknerweichmitteln, Reinigungsmitteln für harte Oberflächen für den Haushalt, Geschirrspülmitteln, Klarspülern, Waschmittelzusätzen, Teppichreinigungsmitteln, Softergent-Mitteln, Weichspülern für einfache Spülung, Ofenreinigungsmitteln, Autowaschmitteln, Transportmittelreinigungsmitteln, Abflussreinigern, Entschäumern, Schaumhemmern, Schaumverstärkern, Antistaubmitteln/Staub abstoßenden Mitteln, industriellen Reinigern, institutionellen Reinigern, hausmeisterlichen Reinigern, Glasreinigern, Graffiti-Entfernern, Betonreinigern, Reinigern für Metall/Maschinenteile, Pestiziden, landwirtschaftlichen Formulierungen und Reinigern für die Gastronomie, Weichmachern, Asphaltzusätzen und -emulgatoren, Reibung reduzierenden Mitteln, Filmbildnern, Rheologiemodifikatoren, Bioziden, Biozidverstärkern, Trennmitteln, Haushaltsreinigungsprodukten, einschließlich flüssiger und pulverförmiger Waschmittel, flüssigen und bahnförmigen Weichspülern, Reinigern für harte und weiche Oberflächen, Sanitärmitteln und Desinfiziermitteln und industriellen Reinigungsprodukten, Emulsionspolymerisation, einschließlich Prozessen für die Herstellung von Latex und zur Verwendungen als Tenside als Benetzungsmittel, inerte Bestandteile von Formulierungen in Pestizidanwendungen oder als Hilfsstoffe, die in Verbindung mit der Abgabe von Pestiziden verwendet werden, einschließlich landwirtschaftlichen Ernteschutzes, Rasen und ornamental, Haus und Garten, und professionellen Anwendungen, institutionellen Reinigungsprodukten, Ölfeldanwendungen, einschließlich ÖI- und Gastransport, Förderung, Stimulierungs- und Bohrchemikalien und Reservoirübereinstimmung und -steigerung, Organotonerden für Bohrschlämme, Spezialschaumbildner für Schaumkontrolle oder -dispergierfähigkeit im Herstellungsprozess von Gips, Zementwandplatten, Betonzusätzen und Feuerbekämpfungsschäumen, Farben und Koalesziermitteln, Farbverdickern und anderen Anwendungen, die ein Kältetoleranzvermögen oder eine Winterisierung erfordern.

13. Zusammensetzung metathesierten natürlichen Öls gemäß Anspruch 1, beinhaltend (i) wobei die Zusammensetzung metathesierten natürlichen Öls ein Molekulargewicht im Zahlenmittel in dem Bereich von 2 600 g/mol bis 3 100 g/mol, ein Molekulargewicht im Gewichtsmittel in dem Bereich von 12 000 g/mol bis 13 500 g/mol und ein z-durchschnittliches Molekulargewicht in dem Bereich von 38 000 g/mol bis 46 000 g/mol und einen Polydispersitätsindex von 4 bis 5 aufweist und wobei ferner die Zusammensetzung metathesierten natürlichen Öls mindestens zweimal metathesiert wurde.

14. Zusammensetzung metathesierten natürlichen Öls gemäß Anspruch 13, wobei die Zusammensetzung metathesierten natürlichen Öls einer Wasserdampfdestillation bei einer Temperatur von zwischen 150 °C und 200 °C unterzogen wird und eine dynamische Viskosität bei 40 °C von zwischen 0,575 Pa·s (575 Centipoise) und 0,67 Pa·s (670 Centipoise) aufweist.

## Revendications

1. Une composition d'huile naturelle ayant subi une métathèse comprenant une huile naturelle ayant subi une métathèse, la composition d'huile naturelle ayant subi une métathèse présentant une masse moléculaire moyenne en nombre comprise dans la gamme allant de 2 000 g/mol à 4 000 g/mol, une masse moléculaire moyenne en poids comprise dans la gamme allant de 7 000 g/mol à 35 000 g/mol, une masse moléculaire moyenne en z comprise dans la gamme allant de 10 000 g/mol à 125 000 g/mol, et un indice de polydispersité de 2 à 12, une viscosité dynamique à 40 °C comprise entre 0,2 Pa•s (200 centipoises) et 3,5 Pa•s (3 500 centipoises), et une viscosité dynamique à 100 °C comprise entre 0,075 Pa•s (75 centipoises) et 0,6 Pa•s (600 centipoises).

2. La composition d'huile naturelle ayant subi une métathèse de la revendication 1, la composition d'huile naturelle ayant subi une métathèse présentant une masse moléculaire moyenne en nombre comprise dans la gamme allant de 2 300 g/mol à 3 400 g/mol, une masse moléculaire moyenne en poids comprise dans la gamme allant de 9 600 g/mol à 31 500 g/mol, et une masse moléculaire moyenne en z comprise dans la gamme allant de 28 000 g/mol à 111 000 g/mol, et un indice de polydispersité de 4 à 12.

3. La composition d'huile naturelle ayant subi une métathèse de la revendication 1, la composition d'huile naturelle ayant subi une métathèse présentant une viscosité dynamique à 40 °C comprise entre 0,55 Pa•s (550 centipoises) et 3,25 Pa•s (3 250 centipoises).

4. La composition d'huile naturelle ayant subi une métathèse de n'importe lesquelles des revendications 1 à 3, la composition d'huile naturelle ayant subi une métathèse étant fabriquée en faisant réagir une matière première huile naturelle en présence d'un catalyseur de métathèse afin de former un premier produit d'huile naturelle ayant subi une métathèse, et dans laquelle en sus le premier produit d'huile naturelle ayant subi une métathèse subit une auto-métathèse afin de former un autre produit d'huile naturelle ayant subi une métathèse.

5. La composition d'huile naturelle ayant subi une métathèse de n'importe lesquelles des revendications 1 à 3, la composition d'huile naturelle ayant subi une métathèse étant fabriquée en faisant réagir une huile naturelle en présence d'un catalyseur de métathèse afin de former un premier produit d'huile naturelle ayant subi une métathèse, et dans laquelle en sus le premier produit d'huile naturelle ayant subi une métathèse subit une métathèse croisée avec une huile naturelle afin de former un autre produit d'huile naturelle ayant subi une métathèse.

6. La composition d'huile naturelle ayant subi une métathèse de n'importe lesquelles des revendications 1 à 3, la composition d'huile naturelle ayant subi une métathèse pouvant être soumise à une extraction à la vapeur à une température comprise entre 100 °C et 250 °C.

7. La composition d'huile naturelle ayant subi une métathèse de n'importe lesquelles des revendications 1 à 3, la composition d'huile naturelle ayant subi une métathèse pouvant être soumise à des conditions de pression réduite afin d'enlever des oléfines en C12 ou plus légères.

8. La composition d'huile naturelle ayant subi une métathèse de n'importe lesquelles des revendications 1 à 3, dans laquelle lorsque la composition d'huile naturelle ayant subi une métathèse comprend un mélange d'oléfines et d'esters, et dans laquelle en sus le mélange d'oléfines et d'esters peut être séparé en oléfines et esters, et par la suite la transestérification des esters en présence d'un alcool afin de former un produit transestérifié.

9. La composition d'huile naturelle ayant subi une métathèse de n'importe lesquelles des revendications 1 à 3, dans laquelle la masse moléculaire de la composition d'huile naturelle ayant subi une métathèse peut être accrue en transestérifiant la composition d'huile naturelle ayant subi une métathèse avec des diesters.

10. La composition d'huile naturelle ayant subi une métathèse de n'importe lesquelles des revendications 1 à 3, dans laquelle la masse moléculaire de la composition d'huile naturelle ayant subi une métathèse peut être accrue en transestérifiant la composition d'huile naturelle ayant subi une métathèse avec des diacides.

11. La composition d'huile naturelle ayant subi une métathèse de n'importe lesquelles des revendications 1 à 3, dans laquelle l'huile naturelle ayant subi une métathèse dans la composition d'huile naturelle ayant subi une métathèse est sélectionnée dans le groupe consistant en l'huile de canola ayant subi une métathèse, l'huile de colza ayant subi une métathèse, l'huile de noix de coco ayant subi une métathèse, l'huile de maïs ayant subi une métathèse, l'huile de coton ayant subi une métathèse, l'huile d'olive ayant subi une métathèse, l'huile de palme ayant subi une métathèse, l'huile d'arachide ayant subi une métathèse, l'huile de carthame ayant subi une métathèse, l'huile de sésame ayant subi une métathèse, l'huile de soja ayant subi une métathèse, l'huile de tournesol ayant subi une métathèse, l'huile de lin ayant subi une métathèse, l'huile de palmiste ayant subi une métathèse, l'huile d'abrasin ayant subi une métathèse, l'huile de jatropha ayant subi une métathèse, l'huile de moutarde ayant subi une métathèse, l'huile de ricin ayant subi une métathèse, l'huile de caméline ayant subi une métathèse, l'huile de tabouret des champs ayant subi une métathèse, des dérivés de ces huiles ayant subi une métathèse, et des mélanges de ceux-ci.

12. La composition d'huile naturelle ayant subi une métathèse de n'importe lesquelles des revendications 1 à 3, la composition d'huile naturelle ayant subi une métathèse pouvant être utilisée indépendamment et/ou incorporée dans diverses formulations et utilisée comme ingrédients fonctionnels, sélectionnés dans le groupe consistant en des substituts de diméthicone, des détergents à lessive, des assouplissants, des émollients, des polymères de fixation capillaire, des modificateurs de rhéologie, des polymères de conditionnement de spécialité, des tensioactifs, des absorbeurs d'UV, des solvants, des humectants, des agents occlusifs, des cosmétiques, des baumes à lèvres, des rouges à lèvres, des produits de coiffage, des produits de soin solaire, des hydratants, des bâtonnets parfumés, des véhicules de parfum, des agents d'amélioration du toucher, des shampooings/après-shampooings, des savons en barre, des savons/savons liquides pour les mains, des bains moussants, des savons liquides pour le corps, des nettoyants visage, des gels douche, des lingettes, des produits nettoyants pour bébé, des crèmes/lotions, des antitranspirants/déodorants, des lubrifiants, des fluides fonctionnels, des carburants et des additifs pour carburant, des additifs pour de tels lubrifiants, fluides fonctionnels et carburants, des plastifiants, des additifs de bitume, des agents réducteurs de frottement, des agents antistatiques dans les industries du textile et du plastique, des agents de flottation, des agents gélifiants, des agents de durcissement époxy, des inhibiteurs de corrosion, des agents mouillants pour pigments, dans des compositions de nettoyage, des plastiques, des revêtements, des adhésifs, des agents d'amélioration du toucher, des agents filmogènes, des modificateurs rhéologiques, des agents de démoulage, des dispersants, des après-shampooings, des hydrotropes, des produits de nettoyage industriels et institutionnels, des applications de champ pétrolier, des agents moussants de gypse, des enduits d'étanchéité, des formulations agricoles, des compositions pour une récupération améliorée du pétrole, des produits solvants, des produits de gypse, des gels, des semi-solides, des détergents, des détergents liquides puissants, des détergents liquides doux, des adoucissants détergents liquides, des formulations antistatiques, des adoucissants pour sèche-linge, des produits de nettoyage de surfaces dures à usage domestique, des agents de lavage de la vaisselle automatique, des produits de rinçage, des additifs de lessive, des nettoyants de tapis, des compositions détergentes adoucissantes, des adoucissants à rinçage unique, des nettoyants pour four, des produits de lavage de voiture, des nettoyants pour moyens de transport, des nettoyants pour canalisations, des démousseurs, des agents anti-moussants, des renforçateurs de mousse, des produits anti-poussière/répulsifs à poussière, des nettoyants industriels, des nettoyants institutionnels, des produits d'entretien, des nettoyants pour vitres, des décapants pour graffitis, des nettoyants pour le béton, des nettoyants de pièces de machines/nettoyants pour métaux, des pesticides, des formulations agricoles et des nettoyants pour la restauration, des plastifiants, des additifs et émulsifiants de bitume, des agents réducteurs de frottement, des agents filmogènes, des modificateurs rhéologiques, des biocides, des potentialisateurs de biocide, des agents de démoulage, des produits d'entretien ménager, notamment des détergents à lessive en poudre et liquides, des adoucissants liquides ou en feuille, des nettoyants de surfaces dures et molles, des assainissants et désinfectants, et des produits de nettoyage industriel, la polymérisation en émulsion, notamment les procédés pour la fabrication de latex et pour une utilisation comme tensioactifs, comme agents mouillants, matières inertes dans des applications de pesticides, ou comme adjuvants utilisés conjointement avec l'administration de pesticides, notamment du gazon de protection de cultures agricoles et des applications ornementales, de la maison et du jardin, et professionnelles, des produits de nettoyage institutionnels, des applications de champ pétrolier, notamment des produits chimiques de transport, de production, de stimulation et de forage de gaz et de pétrole, et la conformité et l'amélioration des réservoirs, des organoargiles pour boues de forage, des agents moussants de spécialité pour la régulation ou la dispersion de la mousse dans le procédé de fabrication de gypse, de panneaux muraux en ciment, des additifs de béton et des mousses extinctrices, des peintures et des agents de coalescence, des épaississants pour peinture, ou d'autres applications nécessitant une bonne tolérance au froid ou une frigélisation.

13. La composition d'huile naturelle ayant subi une métathèse de la revendication 1 comprenant (i), la composition d'huile naturelle ayant subi une métathèse présentant une masse moléculaire moyenne en nombre comprise dans la gamme allant de 2 600 g/mol à 3 100 g/mol, une masse moléculaire moyenne en poids comprise dans la gamme allant de 12 000 g/mol à 13 500 g/mol, et une masse moléculaire moyenne en z comprise dans la gamme allant de 38 000 g/mol à 46 000 g/mol, et un indice de polydispersité de 4 à 5, et la composition d'huile naturelle ayant subi une métathèse ayant en sus subi une métathèse au moins deux fois.

14. La composition d'huile naturelle ayant subi une métathèse de la revendication 13, la composition d'huile naturelle ayant subi une métathèse étant soumise à une extraction à la vapeur à une température comprise entre 150 °C et 200 °C, et présentant une viscosité dynamique à 40 °C comprise entre 0,575 Pa•s (575 centipoises) et 0,67 Pa•s (670 centipoises).
